(19) Europäisches Patentamt
European Patent Office
Office européen des brevets

(11) EP 2 142 672 B1

(12) **EUROPEAN PATENT SPECIFICATION**

(45) Date of publication and mention of the grant of the patent:
**05.09.2012 Bulletin 2012/36**

(21) Application number: **08733027.0**

(22) Date of filing: **31.03.2008**

(51) Int Cl.:
***C12N 15/11*** *(2006.01)* ***C07H 21/02*** *(2006.01)*
***C07H 21/04*** *(2006.01)*

(86) International application number:
**PCT/US2008/058907**

(87) International publication number:
**WO 2008/121963 (09.10.2008 Gazette 2008/41)**

(54) **Compositions and methods for gene silencing**

Zusammensetzungen und Verfahren zum Gen-Silencing

Compositions et procédés pour le silençage de gènes

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.03.2007 US 921032 P**

(43) Date of publication of application:
**13.01.2010 Bulletin 2010/02**

(73) Proprietor: **Rutgers, The State University of New Jersey**
**New Brunswick, NJ 08901-8559 (US)**

(72) Inventors:
- **GORACZNIAK, Rafal**
**Highland Park, NJ 08904 (US)**
- **GUNDERSON, Samuel I.**
**Piscataway, NJ 08854 (US)**

(74) Representative: **Didmon, Mark et al**
**Potter Clarkson LLP**
**Park View House**
**58 The Ropewalk**
**Nottingham NG1 5DD (GB)**

(56) References cited:
**WO-A2-03/095647** **US-A1- 2003 082 149**
**US-A1- 2003 143 732**

- **FORTES PURI ET AL: "Inhibiting expression of specific genes in mammalian cells with 5' end-mutated U1 small nuclear RNAs targeted to terminal exons of pre-mRNA" PROCEEDINGS OF THE NATIONAL ACADEMY OF SCIENCES OF THE UNITED STATES (PNAS), NATIONAL ACADEMY OF SCIENCE, US, vol. 100, no. 14, 8 July 2003 (2003-07-08), pages 8264-8269, XP002481001 ISSN: 0027-8424 DOI: DOI: 10.1073/PNAS.1332669100**
- **WU C ET AL: "Experimental study of inhibition of hepatitis B by dual-target antisense" ZHONGHUA YIXUE ZAZHI - NATIONAL MEDICAL JOURNAL OF CHINA, BEIJING, CH, vol. 81, no. 10, 25 May 2001 (2001-05-25), pages 605-608, XP008086995 ISSN: 0376-2491**
- **HOSSBACH MARKUS ET AL: "Gene silencing with siRNA duplexes composed of target-mRNA-complementary and partially palindromic or partially complementary single-stranded siRNAs." RNA BIOLOGY APR 2006 LNKD-PUBMED:17114944, vol. 3, no. 2, April 2006 (2006-04), pages 82-89, XP002614061 ISSN: 1555-8584**
- **KATO K ET AL: "Hyperstable U1snRNA complementary to the K-ras transcripts induces cell death in pancreatic cancer cells" BRITISH JOURNAL OF CANCER 20021007 GB LNKD- DOI: 10.1038/SJ.BJC.6600563, vol. 87, no. 8, 7 October 2002 (2002-10-07), pages 898-904, XP002614062 ISSN: 0007-0920**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LIU D ET AL: "Stable human immunodeficiency virus type 1 (HIV-1) resistance in transformed CD4+ monocytic cells treated with multitargeting HIV-1 antisense sequences incorporated into U1 snRNA" JOURNAL OF VIROLOGY, THE AMERICAN SOCIETY FOR MICROBIOLOGY, US, vol. 71, no. 5, 1 May 1997 (1997-05-01), pages 4079-4085, XP002514211 ISSN: 0022-538X
- MONTGOMERY ROBERT A ET AL: "Inhibition of fibrillin 1 expression using U1 snRNA as a vehicle for the presentation of antisense targeting sequence" HUMAN MOLECULAR GENETICS, OXFORD UNIVERSITY PRESS, SURREY, vol. 6, no. 4, 1 January 1997 (1997-01-01), pages 519-525, XP002155260 ISSN: 0964-6906 DOI: DOI: 10.1093/HMG/6.4.519
- BECKLEY S A ET AL: "Reduction of target gene expression by a modified U1 snRNA" MOLECULAR AND CELLULAR BIOLOGY, AMERICAN SOCIETY FOR MICROBIOLOGY, WASHINGTON, US, vol. 21, no. 8, 1 April 2001 (2001-04-01), pages 2815-2825, XP002481002 ISSN: 0270-7306 DOI: DOI:10.1128/MCB. 21.8.2815-2825.2001
- GORMAN ET AL: "Restoration of Correct Splicing of Thalassemic ss-Globin Pre-mRNA by modified U1 snRNAs" JOURNAL OF BIOLOGICAL CHEMISTRY, AMERICAN SOCIETY FOR BIOCHEMISTRY AND MOLECULAR BIOLOGY, INC, US, vol. 275, no. 46, 17 November 2000 (2000-11-17), pages 35914-35919, XP002261981 ISSN: 0021-9258 DOI: DOI: 10.1074/JBC.M006259200
- TEMSAMANI J ET AL: "BIOTINYLATED ANTISENSE METHYLPHOSPHONATE OLIGODEOXYNUCLEOTIDES INHIBITION OF SPLICEOSOME ASSEMBLY AND AFFINITY SELECTION OF U1 AND U2 SMALL NUCLEAR RNPS" JOURNAL OF BIOLOGICAL CHEMISTRY, vol. 266, no. 1, 1991, pages 468-472, XP002614083 ISSN: 0021-9258
- GORACZNIAK RAFAL ET AL: "Gene silencing by synthetic U1 Adaptors" NATURE BIOTECHNOLOGY, vol. 27, no. 3, March 2009 (2009-03), XP002614063 ISSN: 1087-0156

**Description**

## FIELD OF THE INVENTION

This invention relates generally to the field of gene silencing. Specifically, the invention provides compositions and methods for regulating the expression of a gene of interest.

## BACKGROUND OF THE INVENTION

It has long been appreciated that gene expression can be regulated at the post-transcriptional level, defined to be the steps between transcription initiation and release of the nascent polypeptide from the ribosome. Antisense based approaches encompass a broad variety of techniques, but have in common an oligonucleotide that is designed to base pair with its complementary target mRNA, or more broadly to any RNA, leading to either degradation of the RNA or impaired function (e.g., impaired translation). Classical antisense approaches were designed to interfere with translation of the target mRNA or induce its degradation via Rnase H. Ribozyme-containing antisense molecules also can induce RNA degradation and have the advantage that they can be turned over (i.e., re-used) to cleave more RNA targets. RNAi-based approaches have proven more successful and involve using siRNA to target the mRNA to be degraded (see, e.g., Novina et al. (2004) Nature 430:161-4). However, some mRNAs are only modestly downregulated (2-fold) by RNAi and others are refractory.

The 3' end processing (also called polyadenylation, poly(A) tail addition, or cleavage and polyadenylation) of nearly all eukaryotic pre-mRNA comprises two steps: (1) cleavage of the pre-mRNA followed by (2) the synthesis of a poly(A) tail at the 3' end of the upstream cleavage product. 3' end formation is essential to mRNA maturation and, in this sense, is as important as transcription initiation for producing a functional mRNA. 3' end formation also functions to enhance transcription termination, transport of the mRNA from the nucleus, and mRNA translation and stability (Eckner et al. (1991) EMBO J., 10:3513-3522 ; Sachs et al. (1993) J. Biol. Chem., 268:22955-8). Defects in mRNA 3' end formation can profoundly influence cell growth, development and function (see, e.g., Zhao et al. (1999) Microbiol. Mol. Biol. Rev., 63:405-445; Proudfoot et al. (2002). Cell 108:501-12).

Cleavage and polyadenylation requires two elements. The highly conserved AAUAAA sequence (also called the poly(A) signal or the hexanucleotide sequence) is found 10 to 30 nucleotides upstream of the cleavage site. This hexanucleotide is essential for both cleavage and polyadenylation and any point mutations (with the exception of AUUAAA) result in a large decrease in its activity (Proudfoot et al. (1976) Nature 263:211-4 ; Sheets et al. (1990) Nucl. Acids Res., 18:5799-805). However, recent bioinformatic studies have suggested that single-base variants or more-rarely double-base variants of AWUAAA (W=A or U) are allowed (Beaudoing et al. (2000) Gen. Res. 10:1001-1010 ; Tian et al. (2005) Nuc. Acids Res., 33:201-12).

The second element is a less-conserved U- or GU-rich region approximately 30 nucleotides downstream of the cleavage site and thus is called the downstream sequence element (DSE). Point mutations or small deletions do not greatly influence DSE's function. Nevertheless, the proximity of the DSE to the poly(A) site can affect the choice of the cleavage site and the efficiency of cleavage (Zhao et al. (1999). Microbiol. Mol. Biol. Rev., 63:405-445). The cleavage site itself (usually referred to as the pA site or poly(A) site) is selected mainly by the distance between the AAUAAA signal and the DSE (Chen et al. (1995). Nuc. Acids Res., 23:2614-2620). For most genes, cleavage happens after a CA dinucleotide.

In addition to the above signals, auxiliary sequences have also been found to have a positive or negative modulatory activity on 3' end processing.

The cleavage/polyadenylation machinery is composed of multiple protein factors with some having multiple subunits. The endonucleolytic cleavage step involves Cleavage/Polyadenylation Specificity Factor (CPSF) binding to A(A/U)UAAA and Cleavage stimulatory Factor (CstF) binding the DSE. Other required factors include Cleavage Factors 1 and 2 (CF $I_m$ and CF $II_m$), RNA polymerase II (Pol II), Symplekin, and poly(A) polymerase (PAP), although the absolute requirement for PAP is still unclear. Once cleavage has occurred the downstream pre-mRNA fragment is rapidly degraded whereas the upstream fragment undergoes poly(A) tail addition that requires CPSF, PAP, and poly(A)-binding protein II (PAB II).

In principle, having the ability to switch on or off a gene's poly(A) site or sites is a way to directly control expression of that gene. There are natural examples where a gene's expression can be controlled by dialing up or down the poly(A) site. Perhaps the best understood example involves excess U1A protein negatively autoregulating its own synthesis by inhibiting polyadenylation of its own pre-mRNA. Without a poly(A) tail, the mRNA fails to leave the nucleus and is degraded leading to lower levels of U1A mRNA and U1A protein. The mechanism involves 2 molecules of U1A protein binding to a site just upstream of its own pre-mRNA's poly(A) site with the resulting $(U1A)_2$-pre-mRNA complex inhibiting 3'-end processing of the U1A pre-mRNA by inhibiting the polyadenylation activity of PAP (Boelens et al. (1993) Cell 72:881-892; Gunderson et al. (1994) Cell 76:531-541; Gunderson et al. (1997) Genes Dev., 11:761-773). An illustrative example, albeit artificial, of "dialing" is found in Guan et al. (Mol. Cell. Biol. (2003) 23:3163-3172). Guan et al. demonstrate that endogenous U1A protein levels are dialed up or down by dialing up or down the activity of its poly(A) site through a stably-expressed epitope-tagged U1A protein that is under the control of a Tet-regulated promoter. The epitope-tagged U1A protein is not subject to autoregulation because its expression cassette lacks the autoregulatory 3'UTR element.

Another natural example of dialing a poly(A) site involves U1 snRNP binding to a "U1 site" just upstream of the poly(A) site of the bovine papillomavirus type 1 (BPV1) late gene pre-mRNA (Furth et al. (1994) Mol. Cell. Biol., 14: 5278-5289). The term "U1 site", which stands for U1 snRNP binding site, is used so as to distinguish it from U1 snRNP's better known function in 5' splice site (5'ss) binding during pre-mRNA splicing. U1 snRNP consists of 10 proteins in complex with a 164 nucleotide U1 snRNA that base pairs to the BPV1 U1 site via nucleotides 2-11 of U1 snRNA (see, e.g., Will et al. (1997) Curr. Opin. Cell Biol., 9:320-8), notably the same nucleotides 2-11 also basepair to the 5'ss sequence as part of the splicing mechanism. Subsequent to its discovery in BPV1, mechanistic studies demonstrated the U1-70K component of the U1 snRNP directly binds to and inhibits the polyadenylation activity of poly(A) polymerase (Gunderson, et al. (1998) Mol. Cell 1:255-264), the enzyme that adds the poly(A) tail. Additional studies in vivo that eliminated the U1-70K binding site confirmed U1-70K as the effector subunit that inhibits expression (Beckley et al. (2001) Mol. Cell Biol., 21:2815-25; Sajic et al. (2007) Nuc. Acids Res., 35:247-55).

The U1in gene silencing technologies use 5'-end-mutated U1 snRNA (see, e.g., U.S. Patent Application Publication Nos. 2003/0082149 and 2005/0043261). U1in stands for U1 snRNP inhibition of expression and refers to two recently developed gene silencing technologies that involve expression of a 5'-end-mutated U1 snRNA where nucleotides 2-11 of U1 snRNA are complementary to a 10 nucleotide sequence in the target gene's 3' terminal exon. The 5'-end-mutated U1 snRNA is expressed from a U1 snRNA expression cassette containing promoter elements and a 3' end formation signal from the U1 snRNA gene. The 5'-end-mutated U1 snRNA transcript assembles with the canonical U1 snRNP proteins into a 5'-end-mutated U1 snRNP that then binds to and inhibits polyadenylation of the targeted pre-mRNA. The 3 key features to make U1in silencing work are: (1) the U1 site on the target pre-mRNA and the 5'-end-mutated U1 snRNA must be perfectly complementary across all 10 basepairs, as a single base mismatch is sufficient to lose silencing (Liu et al. (2002) Nuc. Acids Res., 30:2329-39), (2) the U1 site must be in the 3' terminal exon of the target pre-mRNA (Beckley et al. (2001) Mol. Cell Biol., 21:2815-25; Fortes et al. (2003) Proc. Natl. Acad. Sci., 100: 8264-8269), and (3) the U1-70K binding site on the U1 snRNA must be intact. Although U1in has been successfully used in several instances, its development as a widely-used technology has been limited for a variety of reasons.

In view of the foregoing, it is clear that there is still a need for methods of regulating gene expression.

## SUMMARY OF THE INVENTION

The invention is defined in the accompanying claims. In accordance with the instant invention, nucleic acid molecules for inhibiting the expression of a gene of nucleic are provided, wherein the nucleic acid molecules comprise an annealing domain operably linked to at least one effector domain, wherein the annealing domain hybridizes to the pre-mRNA of the gene of interest and wherein the effector domain hybridizes to the U1 snRNA of U1 snRNP.

In accordance with another aspect of the invention, in vitro methods are provided for inhibiting the expression of a gene of interest comprising delivering to a cell at least one of the nucleic acid molecules of the instant invention.

In accordance with another aspect of the invention, compositions are provided which comprise at least one of the nucleic acid molecules of the invention and at least one pharmaceutically acceptable carrier.

In still another aspect, vectors encoding the nucleic acid molecules of the instant invention are also provided.

## BRIEF DESCRIPTIONS OF THE DRAWING

Figure 1A is a schematic of a U1 adaptor oligonucleotide depicting its 2 domains: an annealing domain to base pair to the target gene's pre-mRNA in the 3' terminal exon and an effector domain that inhibits maturation of the pre-mRNA via binding of endogenous U1 snRNP. The provided sequence of the effector domain is SEQ ID NO: 1. Figure 1B is a schematic of the U1 adaptor annealing to target pre-mRNA. Figure 1C is a schematic of the U1 adaptor binding U1 snRNP, which leads to poly(A) site inhibition. $\psi$ = pseudouridines of the U1 snRNA in the U1 snRNP. The provided sequence of the U1 snRNA in the U1 snRNP is SEQ ID NO: 2.

Figure 2A provides schematics of (1) p717B, comprising a standard Renilla reporter with its 3'UTR and poly(A) signal sequences replaced with those from the human MARK1 gene that has a naturally occurring U1 site (SEQ ID NO: 1), and (2) p717ΔB, which matches p717B except for a 4 nucleotide mutation (lowercase letters in SEQ ID NO: 4) in the U1 site. Relative expression levels of the plasmids upon transient expression in HeLa cells along with a Firefly luciferase control are shown, indicating the wild type U1 site represses expression by 30-fold. Figure 2B provides a schematic of a U1 adaptor inhibiting Renilla luciferase expression. The p717ΔB Renilla reporter with a MARK1 3'UTR having a mutated U1 site (SEQ ID NO: 5) was co-transfected with LNA6 (SEQ ID NO: 6), a U1 adaptor designed to inhibit the poly (A) site via binding of endogenous U1 snRNP (SEQ ID NO: 2). The bold font indicates LNA bases to increase annealing. LNA7 (SEQ ID NO : 7) is a control that matches LNA6 except for mutation of the effector domain. The LNA6 and LNA7 binding site is indicated by the shaded box.

Figure 3A is a graph of the inhibitory activity of LNA6 and LNA7 on the Renilla reporter. Grey bars are LNA6, white bars are LNA7, and the black bar is M13. Figure 3B provides a graph of the inhibitory activity of LNA6 as a function of concentration. Values are normalized to the M13 control oligo. $IC_{50}$ = Inhibitory Concentration needed to achieve 50% inhibition. The bottom curve is the inhibition of p717ΔB and the top curve is the inhibition of the SV40 reporter having the 15 nucleotide isolated LNA6 binding site (gray box in Fig. 3C). Figure 3C provides schematics of p717ΔB and pRL-LNA6.

Figure 4A provides schematics of pRL-LNA6 and pRL-$(LNA6)_2$. Figure 4B provides a graph demonstrating the inhibitory activity of LNA6 U1 adaptor on plasmids containing one or two LNA6 binding sites. Values are normalized to the LNA7 control oligo. The top curve is the inhibition of the pRL-LNA6 plasmid and the bottom curve is the inhibition of the pRL-$(LNA6)_2$ plasmid.

Figure 5A is a schematic of a U1 adaptor (LNA13; SEQ ID NO: 13) designed to target the C-raf-1 pre-mRNA by targeting a 3'UTR sequence. The bold font indicates LNA bases to increase annealing. The U1 snRNP sequence is SEQ ID NO: 2 and the flanking and LNA13 binding site is SEQ ID NO: 12. Figure 5B is a graph depicting the inhibitory activity of varying concentrations of LNA13 on C-raf-1 mRNA as measured by Q-PCR and normalized to GAPDH.

Figure 6A provides a schematic of the Renilla reporter pRL-wtC-Raf-1 (also called p722L) with a C-raf-1 3'UTR and sequences past the poly(A) site, and a graph depicting the $IC_{50}$ values for LNA13 inhibition of pRL-wtC-Raf-1 expression. Figure 6B provides a schematic of plasmid pRL-LNA13 which has a single binding site for LNA13, and a graph depicting the inhibition of expression by LNA13 as a function of concentration.

Figure 7A provides the sequences of LNA6 (SEQ ID NO: 6), LNA17 (SEQ ID NO: 14), Ome-1 (SEQ ID NO: 15), and Ome-5 (SEQ ID NO: 16). Figure 7B provides a graph of the inhibitory activity of 60 nM of adaptors co-transfected with the pRL-wtC-raf-1 plasmid or the pRL-SV40 control plasmid into HeLa cells.

Figure 8A provides the sequences of LNA17 (SEQ ID NO: 14), LNA21 (SEQ ID NO: 17), LNA22 (SEQ ID NO: 18), and LNA23 (SEQ ID NO: 19). Figure 8B provides a graph of the inhibitory activity of adaptor variants of LNA17 having phosphorothioate (PS) bonds and different attachment sites for the U1 domain by co-transfection with pRL-LNA6 (p782J) in HeLa cells.

Figure 9A provides the sequences of LNA17 (SEQ ID NO: 14), LNA24/15 (SEQ ID NO: 20), and LNA24/12 (SEQ ID NO: 21). Figure 9B provides a graph depicting the inhibitory activity of LNA17, LNA24/15, and LNA24/12.

Figure 10 provides a schematic of pRL-wtC-raf-1 and the sequences of LN13 (SEQ ID NO: 13) and LNA25-mtH/U1 (SEQ ID NO: 22). The inhibitory activity of 30 nM of adaptors co-transfected with the pRL-wtC-raf-1 plasmid into HeLa cells is also provided.

Figure 11A provides a schematic of p782J and the sequences of LNA6 (SEQ ID NO: 6), LNA17-13 (SEQ ID NO: 23), LNA17-12 (SEQ ID NO: 24), LNA17-11 (SEQ ID NO: 25), LNA17-10 (SEQ ID NO: 14), LNA17-9 (SEQ ID NO: 26), LNA17-8 (SEQ ID NO: 27), and LNA17-7 (SEQ ID NO: 28). Figure 11B provides a graph of the inhibitory activity of 30 nM of adaptors co-transfected with the pRL-LNA6 plasmid into HeLa cells.

Figure 12 provides a schematic of pRL-LNA6 and a graph depicting the inhibitory activity of LNA17-11 adaptor when transfected into different cells lines. DU145 and PC3 are human cell lines originally derived from more aggressive prostate cancers, whereas LnCap was derived from a less aggressive prostate cancer. SH-SY5Y is a human brain cell line.

Figure 13A provides a graph depicting the inhibition activity with the combination of co-transfected siRNA and U1 adaptors. Figure 13B provides a schematic of pRL-GADPH and the sequence of LNA12 (SEQ ID NO: 29). Figure 13C provides a graph depicting the inhibition activity of the combination of co-transfected siRNA and U1 adaptors.

Figure 14A provides a schematic of p722L and the sequences of LNA-mtH/U1 (SEQ ID NO: 22), LNA25-H/mtU1 (SEQ ID NO: 30), and LNA25-H/U1 (SEQ ID NO: 31). Figure 14B provides a graph depicting the inhibitory activity of the combination in one oligonucleotide of U1 Adaptor activity and Rnase H activity (i.e., traditional antisense design).

Figure 15A provides images of Western blots of 40 μg of total protein extracts from HeLa cells transfected in 6 well plates with 30 nM oligonucleotide. The proteins were separated on 12% SDS-PAGE and probed with mouse cRAF, PARP, and GAPDH antibodies. Figure 15B provides a graph depicting the Q-PCR with C-raf-1-specific primers with the data normalized to GAPDH mRNA.

Figure 16A provides Western blots of transfected HeLa cells lysed in SDS buffer and probed with mouse cRAF and GAPDH antibodies. Figure 16B provides a graph depicting Q-PCR with C-raf-1-specific primers with the data normalized to GAPDH mRNA.

## DETAILED DESCRIPTION OF THE INVENTION

The invention is defined in the accompanying claims.

The methods of the invention comprise the use of the U1 adaptor molecule (see, generally, Figure 1). In its simplest form the U1 adaptor molecule is an oligonucleotide with two domains: (1) an annealing domain designed to base pair to the target gene's pre-mRNA (e.g., in the terminal exon) and (2) an effector domain (also referred to as the U1 domain) that inhibits 3'-end formation of the target pre-mRNA via binding endogenous U1 snRNP. Without being bound by theory, the U1 adaptor tethers endogenous U1 snRNP to a gene-specific pre-mRNA and the resulting complex blocks proper 3' end formation. Notably, U1 snRNP is highly abundant (1 million/mammalian cell nucleus) and in stoichiometric excess compared to other spliceosome components. Therefore, there should be no deleterious effects of titrating out endogenous U1 snRNP.

Preferably, the overall U1 adaptor molecule is resistant to nucleases and is able to enter cells either alone or in complex with delivery reagents (e.g., lipid-based transfection reagents). The U1 adaptor oligo should also be capable of entering the nucleus to bind to pre-mRNA. This property has already been established in those antisense approaches that utilize the Rnase H pathway where the oligo enters the nucleus and binds to pre-mRNA. Additionally, it has been showed that antisense oligos can bind to nuclear pre-mRNA and sterically block access of splicing factors leading to altered splicing patterns (Ittig et al. (2004) Nuc. Acids Res., 32:346-53).

The annealing domain of the U1 adaptor molecule is preferably designed to have high affinity and specificity to the target site on the target pre-mRNA. In a preferred embodiment, a balance should be achieved between having the annealing domain too short, as this will jeopardize affinity, or too long, as this will promote "off-target" effects or alter other cellular pathways. Furthermore, the annealing domain should not interfere with the function of the effector domain (for example, by base pairing and hairpin formation). The U1 adaptor annealing domain does not have an absolute requirement on length. However, the annealing domain will typically be from about 10 to about 50 nucleotides in length, more typically from about 10 to about 30 nucleotides or about 10 to about 20 nucleotides. The annealing domain may be at least 75%, at least 80%, at least 85%, at least 90%, at least 95%, at least 97%, or, more preferably, 100% complementary to the gene of interest. In one embodiment, the annealing domain hybridizes with a target site within the 3' terminal exon, which includes the terminal coding region and the 3'UTR and polyadenylation signal sequences (e.g., through the polyadenylation site). In another embodiment, the target sequence is within about 500 basepair, about 250 basepair, about 100 basepair, or about 50 basepair of the poly(A) signal sequence.

In a particular embodiment, the U1 adaptor may comprise at least one nucleotide analog. The nucleotide analogs may be used to increase annealing affinity, specificity, bioavailability in the cell and organism, cellular and/or nuclear transport, stability, and/or resistance to degradation. For example, it has been well-established that inclusion of (Locked Nucleic Acid) LNA bases within an oligonucleotide increases the affinity and specificity of annealing of the oligonucleotide to its target site (Kauppinen et al. (2005) Drug Discov. Today Tech., 2:287-290; Orum et al. (2004) Letters Peptide Sci., 10:325-334). Unlike RNAi and RNase H-based silencing technologies, U1 adaptor inhibition does not involve enzymatic activity. As such, there is significantly greater flexibility in the permissible nucleotide analogs that can be employed in the U1 adaptor analogs when compared with oligos for RNAi and RNase H-based silencing technologies.

Nucleotide analogs include, without limitation, nucleotides with phosphate modifications comprising one or more phosphorothioate, phosphorodithioate, phosphodiester, methyl phosphonate, phosphoramidate, methylphosphonate, phosphotriester, phosphoroaridate, morpholino, amidate carbamate, carboxymethyl, acetamidate, polyamide, sulfonate, sulfonamide, sulfamate, formacetal, thioformacetal, and/or alkylsilyl substitutions (see, e.g., Hunziker and Leumann (1995) Nucleic Acid Analogues: Synthesis and Properties, in Modern Synthetic Methods, VCH, 331-417; Mesmaeker et al. (1994) Novel Backbone Replacements for Oligonucleotides, in Carbohydrate Modifications in Antisense Research, ACS, 24-39); nucleotides with modified sugars (see, e.g., U.S. Patent Application Publication No. 2005/0118605) and sugar modifications such as 2'-O-methyl (2'-O-methylnucleotides) and 2'-O-methyloxyethoxy; and nucleotide mimetics such as, without limitation, peptide nucleic acids (PNA), morpholino nucleic acids, cyclohexenyl nucleic acids, anhydrohexitol nucleic acids, glycol nucleic acid, threose nucleic acid, and locked nucleic acids (LNA) (see, e.g., U.S. Patent Application Publication No. 2005/0118605). See also U.S. Patent Nos. 5,886,165; 6,140,482; 5,693,773; 5,856,462; 5,973,136; 5,929,226; 6,194,598; 6,172,209; 6,175,004; 6,166,197; 6,166,188; 6,160,152; 6,160,109; 6,153,737; 6,147,200; 6,146,829; 6,127,533; and 6,124,445.

In a particular embodiment, the U1 domain of the U1 adaptor binds with high affinity to U1 snRNP. The U1 domain may hybridize with U1 snRNA (particularly the 5'-end and more specifically nucleotides 2-11) under moderate stringency conditions, preferably under high stringency conditions, and more preferably under very high stringency conditions. In another embodiment, the U1 domain is perfectly complementary to nucleotides 2-11 of endogenous U1 snRNA. Therefore, the U1 domain may comprise the sequence 5'-CAGGUAAGUA-3' (SEQ ID NO: 1). In another embodiment, the U1 domain is at least 70%, at least 75%, at least 80%, at least 85%, and more preferably at least 90%, at least 95%, or at least 97% homologous to SEQ ID NO: 1. The U1 domain may comprise additional nucleotides 5' or 3' to SEQ ID NO: 1. For example, the U1 domain may comprise at least 1, 2, 3, 4, 5, or up to 10 or 20 nucleotides 5' or 3' to SEQ ID NO: 1. Indeed, as demonstrated hereinbelow, increasing the length of the U1 domain to include basepairing into stem 1 and/or basepairing to position 1 of U1 snRNA improves the U1 adaptor's affinity to U1 snRNP. The effector domain may be from about 8 nucleotides to about 30 nucleotides, from about 10 nucleotides to about 20 nucleotides, or from about 10 to about 15 nucleotides in length.

The insertion of point mutations into the U1 domain, i.e., diverging from the consensus sequence SEQ ID NO: 1, can moderate silencing. Indeed, altering the consensus sequence will produce U1 domains of different strength and affinity for the U1 snRNA, thereby leading to different levels of silencing. Therefore, once an annealing domain has been determined for a gene of interest, different U1 domains of different strength can be attached to the annealing domain to effect different levels of silencing of the gene of interest. For example gAGGUAAGUA (SEQ ID NO: 3) would bind more weakly to U1 snRNP than SEQ ID NO: 1 and, therefore, would produce a lower level of silencing. As discussed above, nucleotide analogues can be included in the U1 domain to increase the affinity to endogenous U1 snRNP. The addition of nucleotide analogs may not be considered a point mutation if the nucleotide analog binds the same nucleotide as the replaced nucleotide.

Notably, care should be taken so as to not design a U1 adaptor wherein the effector domain has significant affinity for the target site of the mRNA or the sites immediately flanking the target site. In other words, the target site should be selected so as to minimize the base pairing potential of the effector domain with the target pre-mRNA, especially the portion flanking upstream of the annealing site.

To increase the silencing ability of the U1 adaptors, the U1 adaptor should also be designed to have low self annealing so as to prevent the formation of hairpins within a single U1 adaptor and/or the formation of homodimers or homopolymers between two or more U1 adaptors.

The annealing and effector domains of the U1 adaptor may be linked such that the effector domain is at the 5' end and/or 3' end of the annealing domain. Further, the annealing and effector domains may be operably linked via a linker domain. The linker domain may comprise 1, 2, 3, 4, 5, 6, 7, 8, 9, 10, up to 15, up to 20, or up to 25 nucleotides.

In another embodiment of the instant invention, more than one U1 adaptor directed to a gene of interest may be used to modulate expression. As shown hereinbelow, multiple U1 adaptors targeting (annealing) to different sequences in the same pre-mRNA should give enhanced inhibition (as has already been shown in Figure 9).

In still another embodiment, the U1 adaptor can be combined with other methods of modulating the expression of a gene of interest. For example, a U1 adaptor can be used in coordination with antisense approaches such as, RNase H-based methods, RNAi, and morpholino-based methods to give enhanced inhibition. Inasmuch as U1 adaptors utilize a different mechanism than antisense approaches, the combined use will result in an increased inhibition of gene expression compared to the use of a single inhibitory agent alone. Indeed, U1 adaptors may target the biosynthetic step in the nucleus whereas RNAi and certain antisense approaches generally target cytoplasmic stability or translatability of a pre-existing pool of mRNA.

The U1 adaptors of the instant invention may be administered in vitro to a cell via an expression vector. For example, a U1 adaptor can be expressed from a vector such as a plasmid or a virus. Expression of such short RNAs from a plasmid or virus has become routine and can be easily adapted to express a U1 adaptor.

Also described herein but not claimed is an approach in which the effector domain of the U1 adaptor can be replaced with the binding site for any one of a number of nuclear factors that regulate gene expression. For example, the binding site for polypyrimidine tract binding protein (PTB) is short and PTB is known to inhibit poly(A) sites. Thus, replacing the effector domain with a high affinity PTB binding site would also silence expression of the target gene.

There are U1 snRNA genes that vary in sequence from the canonical U1 snRNA described hereinabove. Collectively, these U1 snRNA genes can be called the U1 variant genes. Some U1 variant genes are described in GenBank Accession Nos. L78810, AC025268, A025264 and AL592207 and in Kyriakopoulou et al. (RNA (2006) 12: 1603-11), which identified close to 200 potential U1 snRNA-like genes in the human genome. Since some of these these U1 variants have a 5' end sequence different than canonical U1 snRNA, one plausible function is to recognize alternative splice signals during pre-mRNA splicing. Accordingly, the U1 domain of the U1 adaptors of the instant invention may be designed to hybridize with the 5' end of the U1 variant snRNA in the same way as the U1 domain was designed to hybridize with the canonical U1 snRNA as described herein. The U1 adaptors which hybridize to the U1 variants may then be used to modulate the expression of a gene of interest.

There are many advantages of the U1 adaptor technology to other existing silencing technologies. Certain of these advantages are as follows. First, the U1 adaptor separates into two independent domains: (1) the annealing (i.e., targeting) activity and (2) the inhibitory activity, thereby allowing one to optimize annealing without affecting the inhibitory activity or vice versa. Second, as compared to other technologies, usage of two adaptors to target the same gene gives additive even synergistic inhibition. Third, the U1 adaptor has a novel inhibitory mechanism. Therefore, it should be compatible when used in combination with other methods. Fourth, the U1 adaptor inhibits the biosynthesis of mRNA by inhibiting the critical, nearly-universal, pre-mRNA maturation step of poly(A) tail addition (also called 3' end processing).

Although U1in has been successfully used by in certain circumstances, its development as a widely-used technology has been limited for a variety of reasons. Certain of these reasons are described as follows.

First, for U1in, there is a possibility that off-target silencing will occur because a 10 nucleotide sequence, even if it is restricted to the terminal exon, may not be long enough to be unique in the human transcriptome or in most vertebrate transcriptomes. The U1 adaptor annealing domain does not have restrictions in length or nucleotide composition (e.g., nucleotide analogues may be used) and so a length of 15 nucleotides, such as was used for LNA6 described hereinbelow, is sufficient for uniqueness in a typical mammalian transcriptome.

Second, for U1in, inhibition is readily negated if the pre-mRNA target sequence is "buried" within intramolecular RNA secondary structure. Indeed, if just half of the 10 nucleotide target (i.e., 5 nucleotides) is base paired, then that is sufficient to block binding of the 5'-end-mutated U1 snRNP as well as endogenous U1 snRNP (Fortes et al. (2003) Proc. Natl. Acad. Sci., 100:8264-8269; Abad et al. Requirements for gene silencing mediated by U1snRNA binding to a target sequence. Nucleic Acids Res. (2008) in press, epub February 25, 2008). This important problem of target accessibility is not easily solved and is due in large part to the well-recognized difficulty algorithms have in accurately predicting short mRNA secondary structures. The U1 adaptor effector domain is not masked by RNA structure because it is designed to not base pair with the target pre-mRNA.

Third, for U1in, the 5'-end-mutated U1 snRNA is too long to synthesize as an oligonucleotide and attempts to shorten it while maintaining activity have failed. Thus, 5'-end-mutated U1 snRNA can only be expressed from DNA (e.g., from a plasmid or viral delivery system) that has a suitable U1 snRNA expression cassette. The U1 adaptor is an oligonucleotide that does not have a length restriction and typically is in the range of 20-30 nucleotides in length.

Fourth, for U1in, inhibition by transient transfection of a 5'-end-mutated U1 snRNA plasmid is often inefficient because U1 snRNA maturation takes up to 18 hours, thereby resulting in a significant delay or "lag time" in accumulation of the inhibitory complex, leading to a delay in inhibition of the target gene. The U1 adaptor does not have such a lag time.

Fifth, for U1in, an additional potential concern is that "off-target" effects could arise from basepairing of a 5'-end-mutated U1 snRNP to an internal exon or intron that then alters the splicing pattern or affects other steps in the life of that gene's mRNA. This is exacerbated by the fact that the 10 nucleotide targeting sequence for U1in is so short. This concern is mitigated for the U1 adaptor because it has a much longer targeting sequence that can also be readily altered.

Sixth, the expression levels of the 5' end mutated U1 snRNA (the inhibitory molecule for U1in) are significantly lower than the level of endogenous U1 snRNA (1 million molecules/nucleus) which is the corresponding inhibitory molecule for the U1 adaptor technology. Thus, inhibitory levels of the U1 adaptor should be higher.

*Definitions*

"Nucleic acid" or a "nucleic acid molecule" as used herein refers to any DNA or RNA molecule, either single or double stranded and, if single stranded, the molecule of its complementary sequence in either linear or circular form. In discussing nucleic acid molecules, a sequence or structure of a particular nucleic acid molecule may be described herein according to the normal convention of providing the sequence in the 5' to 3' direction. With reference to nucleic acids of the invention, the term "isolated nucleic acid" is sometimes used. This term, when applied to DNA, refers to a DNA molecule that is separated from sequences with which it is immediately contiguous in the naturally occurring genome of the organism in which it originated. For example, an "isolated nuclei acid" may comprise a DNA molecule inserted into a vector, such as a plasmid or virus vector, or integrated into the genomic DNA of a prokaryotic or eukaryotic cell or host organism.

When applied to RNA, the term "isolated nucleic acid" may refer to an RNA molecule encoded by an isolated DNA molecule as defined above. Alternatively, the term may refer to an RNA molecule that has been sufficiently separated from other nucleic acids with which it would be associated in its natural state (i.e., in cells or tissues). An isolated nucleic acid (either DNA or RNA) may further represent a molecule produced directly by biological or synthetic means and separated from other components present during its production.

With respect to single stranded nucleic acids, particularly oligonucleotides, the term "specifically hybridizing" refers to the association between two single-stranded nucleotide molecules of sufficiently complementary sequence to permit such hybridization under pre-determined conditions generally used in the art (sometimes termed "substantially complementary"). In particular, the term refers to hybridization of an oligonucleotide with a substantially complementary sequence contained within a single-stranded DNA molecule of the invention, to the substantial exclusion of hybridization of the oligonucleotide with single-stranded nucleic acids of non-complementary sequence. Appropriate conditions enabling specific hybridization of single stranded nucleic acid molecules of varying complementarity are well known in the art.

For instance, one common formula for calculating the stringency conditions required to achieve hybridization between nucleic acid molecules of a specified sequence homology is set forth below (Sambrook et al., 1989):

$$T_m = 81.5°C + 16.6 \text{Log}\,[Na+] + 0.41(\%\ G+C) - 0.63\ (\%\ \text{formamide}) - 600/\#\text{bp in duplex}$$

As an illustration of the above formula, using [Na+] = [0.368] and 50% formamide, with GC content of 42% and an average probe size of 200 bases, the Tm is 57°C. The Tm of a DNA duplex decreases by 1-1.5°C with every 1% decrease in homology. Thus, targets with greater than about 75% sequence identity would be observed using a hybridization temperature of 42°C.

The stringency of the hybridization and wash depend primarily on the salt concentration and temperature of the solutions. In general, to maximize the rate of annealing of the oligonucleotide with its target, the hybridization is usually carried out at salt and temperature conditions that are 20-25°C below the calculated Tm of the hybrid. Wash conditions should be as stringent as possible for the degree of identity of the probe for the target. In general, wash conditions are selected to be approximately 12-20°C below the Tm of the hybrid. In regards to the nucleic acids of the current invention, a moderate stringency hybridization is defined as hybridization in 6X SSC, 5X Denhardt's solution, 0.5% SDS and 100 μg/ml denatured salmon sperm DNA at 42°C, and washed in 2X SSC and 0.5% SDS at 55°C for 15 minutes. A high stringency hybridization is defined as hybridization in 6X SSC, 5X Denhardt's solution, 0.5% SDS and 100 μg/ml denatured salmon sperm DNA at 42°C, and washed in 1X SSC and 0.5% SDS at 65°C for 15 minutes. A very high stringency hybridization is defined as hybridization in 6X SSC, 5X Denhardt's solution, 0.5% SDS and 100 μg/ml denatured salmon sperm DNA at 42°C, and washed in 0.1X SSC and 0.5% SDS at 65°C for 15 minutes.

The term "primer" as used herein refers to a DNA oligonucleotide, either single stranded or double stranded, either derived from a biological system, generated by restriction enzyme digestion, or produced synthetically which, when placed in the proper environment, is able to functionally act as an initiator of template-dependent nucleic acid synthesis. When presented with an appropriate nucleic acid template, suitable nucleoside triphosphate precursors of nucleic acids, a polymerase enzyme, suitable cofactors and conditions such as a suitable temperature and pH, the primer may be extended at its 3' terminus by the addition of nucleotides by the action of a polymerase or similar activity to yield a primer extension product. The primer may vary in length depending on the particular conditions and requirement of the application. For example, in diagnostic applications, the oligonucleotide primer is typically 15-25 or more nucleotides in length. The primer must be of sufficient complementarity to the desired template to prime the synthesis of the desired extension product, that is, to be able anneal with the desired template strand in a manner sufficient to provide the 3' hydroxyl moiety of the primer in appropriate juxtaposition for use in the initiation of synthesis by a polymerase or similar enzyme. It is not required that the primer sequence represent an exact complement of the desired template. For example, a non complementary nucleotide sequence may be attached to the 5' end of an otherwise complementary primer. Alternatively, non complementary bases may be interspersed within the oligonucleotide primer sequence, provided that the primer sequence has sufficient complementarity with the sequence of the desired template strand to functionally provide a template primer complex for the synthesis of the extension product.

Polymerase chain reaction (PCR) has been described in U.S. Patent Nos: 4, 683, 195, 4,800,195, and 4, 965, 188, the entire disclosures of which are incorporated by reference herein.

The terms "percent similarity", "percent identity" and "percent homology", when referring to a particular sequence, are used as set forth in the University of Wisconsin GCG software program.

A "replicon" is any genetic element, for example, a plasmid, cosmid, bacmid, phage or virus, which is capable of replication largely under its own control. A replicon may be either RNA or DNA and may be single or double stranded.

A "vector" is a genetic element, such as a plasmid, cosmid, bacmid, phage or virus, to which another genetic sequence or element (either DNA or RNA) may be attached. The vector may be a replicon so as to bring about the replication of the attached sequence or element.

An "expression operon" refers to a nucleic acid segment that may possess transcriptional and translational control sequences, such as promoters, enhancers, translational start signals (e.g., ATG or AUG codons), polyadenylation signals, terminators, and the like, and which facilitate the expression of a nucleic acid or a polypeptide coding sequence in a host cell or organism. An "expression vector" is a vector which facilitates the expression of a nucleic acid or a polypeptide coding sequence in a host cell or organism.

The term "oligonucleotide," as used herein, refers to nucleic acid sequences, primers, and probes of the present invention, and is defined as a nucleic acid molecule comprised of two or more ribo or deoxyribonucleotides, preferably more than three. The exact size of the oligonucleotide will depend on various factors and on the particular application and use of the oligonucleotide.

The phrase "small, interfering RNA (siRNA)" refers to a short (typically less than 30 nucleotides long, more typically between about 21 to about 25 nucleotides in length) double stranded RNA molecule. Typically, the siRNA modulates the expression of a gene to which the siRNA is targeted. The term "short hairpin RNA" or "shRNA" refers to an siRNA precursor that is a single RNA molecule folded into a hairpin structure comprising an siRNA and a single stranded loop portion of at least one, typically 1-10, nucleotide.

The term "RNA interference" or "RNAi" refers generally to a sequence-specific or selective process by which a target molecule (e.g., a target gene, protein or RNA) is downregulated via a double-stranded RNA. The double-stranded RNA structures that typically drive RNAi activity are siRNAs, shRNAs, microRNAs, and other double-stranded structures that can be processed to yield a small RNA species that inhibits expression of a target transcript by RNA interference.

The term "antisense" refers to an oligonucleotide having a sequence that hybridize to a target sequence in an RNA by Watson-Crick base pairing, to form an RNA:oligonucleotide heteroduplex with the target sequence, typically with an mRNA. The antisense oligonucleotide may have exact sequence complementarity to the target sequence or near complementarity. These antisense oligonucleotides may block or inhibit translation of the mRNA, and/or modify the processing of an mRNA to produce a splice variant of the mRNA. Antisense oligonucleotides are typically between about 5 to about 100 nucleotides in length, more typically, between about 7 and about 50 nucleotides in length, and even more typically between about 10 nucleotides and about 30 nucleotides in length.

The term "substantially pure" refers to a preparation comprising at least 50-60% by weight of a given material (e.g., nucleic acid, oligonucleotide, protein, etc.). More preferably, the preparation comprises at least 75% by weight, and most preferably 90-95% by weight of the given compound. Purity is measured by methods appropriate for the given compound (e.g. chromatographic methods, agarose or polyacrylamide gel electrophoresis, HPLC analysis, and the like).

The term "gene" refers to a nucleic acid comprising an open reading frame encoding a polypeptide, including both exon and (optionally) intron sequences. The nucleic acid may also optionally include non coding sequences such as promoter or enhancer sequences. The term "intron" refers to a DNA sequence present in a given gene that is not translated into protein and is generally found between exons.

The phrase "operably linked", as used herein, may refer to a nucleic acid sequence placed into a functional relationship with another nucleic acid sequence. Examples of nucleic acid sequences that may be operably linked include, without limitation, promoters, transcription terminators, enhancers or activators and heterologous genes which when transcribed and, if appropriate to, translated will produce a functional product such as a protein, ribozyme or RNA molecule.

"Pharmaceutically acceptable" indicates approval by a regulatory agency of the Federal government or a state government. "Pharmaceutically acceptable" agents may be listed in the U.S. Pharmacopeia or other generally recognized pharmacopeia for use in animals, and more particularly in humans.

A "carrier" refers to, for example, a diluent, preservative, solubilizer, emulsifier, adjuvant, excipient, auxilliary agent or vehicle with which an active agent of the present invention is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous saline solutions and aqueous dextrose and glycerol solutions may be employed as carriers. Suitable pharmaceutical carriers are described in "Remington's Pharmaceutical Sciences" by E.W. Martin.

*Compositions and Methods*

Compositions of the instant invention comprise at least one U1 adaptor of the instant invention and at least one pharmaceutically acceptable carrier. The compositions may further comprise at least one other agent which inhibits the expression of the gene of interest. For example, the composition may further comprise at least one siRNA or antisense oligonucleotide directed against the gene of interest.

The U1 adaptors of the present invention may be administered alone, as naked polynucleotides, to cells in vitro. The U1 adaptor may be administered with an agent which enhances its uptake by cells. In a particular embodiment, the U1 adaptor may be contained within a liposome or polymeric composition (see, e.g., U.S. Patent Nos. 4,897,355; 4,399,498; 4,235,871; 4,231,877; 4,224,179; 4,753,788; 4,673,567; 4, 247, 411; 4, 814, 270; 5, 567, 434; 5, 552, 157; 5, 565, 213; 5,738,868; 5,795,587; 5,922,859; and 6, 077,663, Behr (1994) Bioconjugate Chem. 5:382-389, and Lewis et al. (1996) PNAS 93:3176-3181).

In another embodiment, the U1 adaptor may be delivered to a cell in vitro, in an expression vector such as a plasmid or viral vector. Expression vectors for the expression of RNA molecules preferably employ a strong promoter which may be constitutive or regulated. Such promoters are well known in the art and include, but are not limited to, RNA polymerase II promoters, the T7 RNA polymerase promoter, and the RNA polymerase III promoters U6 and H1 (see, e.g., Myslinski et al. (2001) Nucl. Acids Res., 29:2502-09). Viral-mediated delivery includes the use of vectors based on, without limitation, retroviruses, adenoviruses, adeno-associated viruses, vaccinia virus, lentiviruses, polioviruses, and herpesviruses.

The pharmaceutical compositions of the present invention can be administered by any suitable route, for example, by injection (e.g., intravenously and intramuscularly), by oral, pulmonary, nasal, rectal, or other modes of administration. The compositions can be administered for the treatment of a disease which can be treated through the downregulation of a gene(s). The compositions may be used *in vitro*, *in vivo,* and/or *ex vivo*.

Also described herein but not claimed are kits comprising the compositions, U1 adaptors, and/or vectors of the instant invention.

The following examples describe illustrative methods of practicing the instant invention and are not intended to limit the scope of the invention in any way.

## EXAMPLE I

The following methods were used in the Examples II-VIII.

Cultured cells (typically HeLa cells) were grown in media as recommended by ATCC and seeded the day before transfection such that they would be approximately 50% confluent on the day of transfection. For 24-well-plates, mix #1 and #2 were incubated 15 minutes at room temperature and then gently mixed together and incubated another 20 minutes at room temperature. Mix #1 was made by adding oligos (adaptors, siRNAs, and M13) and reporter plasmids to 50 μl OPTIMEM® media (Invitrogen catalog 51985; Carlsbad, CA). Mix #2 was made by adding 1.8 μl LIPO-FECTAMINE™-2000 (Invitrogen) to 50 μl OPTIMEM® media. The media on the cells in the 24 well dish was removed and 400 μl of fresh complete media was added. Then all of the Mix 1+2 solution (approximately 110 μl) was added to the cells. For 12-well and 6-well plate transfections, the values listed above were scaled up 2-fold and 4-fold, respectively. For luciferase assays, the cells were harvested after 24 hours or 48 hours and luciferase measured using the Promega dual luciferase kit (Madison, WI) and a Turner BioSystems Luminometer (Sunnyvale, CA). For inhibition of endogenous genes, the cells were harvested after 24 or up to 48 hours and either lysed in SDS buffer for Western blotting or total RNA made using a Qiagen RNeasy kit (Valencia, CA).

Enhanced chemiluminescence (ECL) Western blotting was done as previously described (Gunderson et al. (1997) Genes and Dev., 11:761-773; Gunderson et al. (1998) Mol. Cell 1:255-264). Anti-GAPDH antibody (1:10000 dilution; Chemicon; Temecula, CA), a 1:1000 dilution anti-C-raf-1 antibody (R1912 from BD Biosciences; San Jose, Ca) and a 1:1000 dilution for the anti-PARP antibody (Ab-2 from Oncogene; La Jolla, CA) were used. The secondary antimouse and anti-rabbit antibodies were used at a 1:5000 dilution and were obtained from Amersham (Piscataway, NJ) as was the chemiluminescent reagent. The membrane used was Immobilon-P from Millipore (Bedford, MA) and was treated as per manufacturer's instructions.

RNA from transfected cells was isolated using the Rneasy kit from Qiagen. Complimentary DNA was synthesized using 1 μg of RNA, random hexamers, and Moloney Murine Leukemia Virus (MMLV) reverse transcriptase as suggested by the manufacturer (Promega). 50 ng of cDNA was analyzed on real-time PCR using a ROTOR-GENE™ 3000 real time rotary analyzer (Corbett Research; Cambridgeshire, United Kingdom) and QuantiTech SYBR Green PCR kit (Qiagen). Amplification of glyceraldehyde-3-phosphate dehydrogenase (GAPDH) was used as an endogenous control to standardize the amount of sample added to the reaction. The comparative cycle threshold (CT) method was used to analyze the data by generating relative values of the amount of target cDNA. To obtain relative values, the following arithmetic formula was used: $2^{-\Delta\Delta CT}$, where $\Delta$CT= difference between the threshold cycles of the target (c-Raf) and an endogenous reference (GAPDH), and -$\Delta\Delta$CT = difference between $\Delta$CT of the target sample and a control (cells treated with M13 oligo).

## EXAMPLE II

To facilitate testing of the U1 adaptor, the dual luciferase reporter system from Promega was employed where Firefly luciferase was used as a co-transfected control and Renilla luciferase was targeted for inhibition by the U1 adaptor. Plasmid p717B (Figure 2A) was constructed by taking a Promega Renilla luciferase plasmid (pRL-SV40) and replacing its 3'UTR and poly(A) signal sequence with sequences from the human Microtubule Affinity Regulating Kinase (MARK1) 3'UTR and poly(A) signal region including 146 basepairs past the poly(A) site. The human MARK1 3'UTR has a naturally occurring wild type (wt) 10 nucleotide U1 site that is also found in other MARK1 homologs in other vertebrates. The MARK1 wt U1 site in p717B is functional for inhibiting expression. Furthermore, the introduction of a 4 nucleotide mutation in the wt U1 site, thereby producing plasmid p717ΔB, resulted in an approximate 30-fold increase in Renilla expression (see Figure 2A). p717ΔB was tested as it would allow for the comparison of "trans-inhibition" mediated by a U1 adaptor: U1 snRNP complex with the "cis-inhibition" mediated by the MARK1 wt U1 site:U1 snRNP complex.

To target p717AB for inhibition, a U1 adaptor (Figure 2B) called LNA6 that contains a mixture of Locked Nucleic Acid (LNA) nucleotides and phosphoramidate modified bases was used. In theory, any inhibitory activity seen with LNA6 could be due to a combination of two or more activities, namely: (1) the binding of U1 snRNP and (2) traditional antisense effects from its annealing domain, thereby having nothing to do with the effector domain. To distinguish between these activities and facilitate interpretation of the results, a control U1 adaptor called LNA7 was used that matches LNA6 except it is unable to bind endogenous U1 snRNP because of a mutation in the effector domain. Any inhibitory activity seen with LNA7 would arise solely from the action of its annealing domain (antisense activity). Therefore, any observed inhibitory activity with LNA6 that was greater than that observed with LNA7 could be attributed to the binding of U1 snRNP to the effector domain. In other words, comparison of the inhibitory activity of LNA6 with LNA7 would indicate how much inhibition is due to endogenous U1 snRNP binding the effector domain versus inhibition arising solely from traditional antisense effects (e.g., inhibition of translation) that are due to the annealing activity of the annealing domain.

LNA6 was co-transfected with p717ΔB and the control Firefly reporter into HeLa cells and after 24 hours the cells were harvested and luciferase activity measured as per the manufacturer's protocol. Parallel experiments were done where LNA7 was used in place of LNA6. To keep the amount of transfected oligonucleotide constant, an unrelated primer oligonucleotide (the M13 DNA oligonucleotide) was added, where necessary, so that the final amount of total oligonucleotide was held constant at 62 nM. As seen in Figure 3A, LNA6 gives a dose dependent inhibitory activity and this activity is far higher than that of LNA7. Indeed, testing of higher concentrations of LNA7 (>62 nM) indicated its inhibitory activity is approximately the same as that of the M13 DNA oligonucleotide. Therefore, it is evident that nearly all of the inhibitory activity of LNA6 is due to the action of the effector domain rather than antisense effects just from the annealing domain.

In Figure 3B, the inhibitory activity of LNA6 is plotted as a function of its concentration and this allows for the calculation of $IC_{50}$ values, i.e., the concentration of oligo needed to achieve 50% inhibition of expression. The $IC_{50}$ value for LNA6 is 6.35 nM. The $IC_{50}$ values were calculated from 3 independent transfections that were plotted as a function of the U1 adaptor concentration (in this case LNA6) and fitted with a sigmoidal dose-response function using GrapPad Prism software.

Given that the MARK1 3'UTR contains a natural U1 site it was possible that MARK1 sequences flanking the LNA6 binding site contribute to LNA6's inhibitory activity. To test this, the LNA6 binding site was analyzed in isolation by inserting its binding site into pRL-SV40, which has its 3'UTR and poly(A) signal sequences derived from SV40, which is unrelated to MARK1. This plasmid is called pRL-LNA6 (Figure 3C). The co-transfection experiments and analysis above were repeated where pRL-LNA6 was substituted for p717ΔB. It was determined that the $IC_{50}$ value is 6.86 nM which is statistically similar to that seen for the p717ΔB plasmid (Figure 3B). This demonstrates that the LNA6 binding site is necessary and sufficient to confer LNA6's inhibitory activity to the reporter plasmid.

In additional examples, the time of transfection was varied from 24 to 48 hours and the amount of transfected plasmid was also varied. Similar results to those presented above were obtained.

For each targeting site, it may be desirable to optimize the U1 adaptor design in terms of the number and position of LNA bases so as to give optimal inhibition while minimizing off target effects.

It has previously been demonstrated that two U1 snRNP binding sites gave synergistic enhanced inhibition when inserted into the 3' terminal exon of a reporter gene (Fortes et al. (2003) Proc. Natl. Acad. Sci., 100:8264-8269). It has also been shown that two 5'-end-mutated U1 snRNAs gave synergistic inhibition when targeting a single endogenous gene (Fortes et al. (2003) Proc. Natl. Acad. Sci., 100:8264-8269). To demonstrate whether U1 adaptors would behave in the same way, a second LNA6 binding site was inserted into the pRL-LNA6 plasmid to make the pRL-$(LNA6)_2$ plasmid (Figure 4A). The analysis described hereinabove was repeated and the results indicate multiple U1 adaptors give enhanced inhibition (Figure 4B). Notably, such increases in inhibition are rarely seen when targeting one mRNA with two siRNAs (Elbashir et al. (2001) Nature 411:494-8 Novina et al. (2004) Nature 430:161-4).

**EXAMPLE III**

The human C-raf-1 gene was selected to test the U1 adaptor on an endogenous gene. It is well known that the accessibility of the target sequence is often a rate-limiting step in antisense- and siRNA-based approaches. This may also be true for U1 adaptors given their annealing domains have to target pre-mRNA. Given that antisense-based approaches also target nuclear pre-mRNA (although for Rnase H-mediated degradation and not poly(A) site inhibition), it was reasoned that a successful antisense oligonucleotide would imply the target pre-mRNA is available for annealing and could be targeted with a U1 adaptor at the same or a nearby sequence. Monia et al. (Nat Med. (1996) 2:668-75) screened ~34 antisense oligonucleotides to determine which would be best at inhibiting expression of the C-raf-1 kinase gene. Of the 34, only 2 good inhibitor were found. Both antisense oligonucleotides were in the terminal exon and are, therefore, candidates for targeting with a U1 adaptor.

C-raf-1 is a member of the raf family of genes that are downstream effectors of ras protein function as part of the MAP kinase signaling pathway (GenBank Accession No. NM_002880). Mutations in raf genes transform cells in vitro and are associated with certain tumors. High expression of C-raf-1 mRNA and protein is also found in certain tumors.

As diagrammed in Figure 5A, a sequence in the 3'UTR of the endogenous human C-raf-1 gene was targeted with LNA13. Notably, the U1 domain composition is different between LNA6 and LNA13 in that the positions of the LNAs were changed. This was done in part to avoid intramolecular basepairing (e.g., hairpin formation) and intermolecular basepairing (e.g., oligomerization) interactions of the LNA13 adaptor as predicted by computational methods using freely available algorithms such as those from IDT Corporation (Iowa) or Exiqon (Denmark).

HeLa cells are known to express C-raf-1 mRNA and quantitative PCR (Q-PCR) conditions were established to measure mRNA levels of C-raf-1, GAPDH, and Actin. Transfection conditions were as described above with the M13 oligonucleotide being used to bring the final concentration of total oligonucleotide to 62 nM. After 24 hours cells were harvested and used to make total RNA. Q-PCR conditions were: 95° melt, 55° anneal, and 72° extend for 15 seconds. The primers used were: C-raf-1 forward primer = 5'-TGTTTCCAGGATGCCTGTT-3' (SEQ ID NO: 8), C-raf-1 reverse primer = 5'-GGACATTAGGTGTGGATGTCG-3' (SEQ ID NO: 9), GAPDH forward primer = 5'-AGCCACATCGCTCAGACAC-3' (SEQ ID NO: 10), and GAPDH reverse primer = 5'-GCCCAATACGACCAAATCC-3' (SEQ ID NO: 11).

Q-PCR was performed using a ROTOR-GENE™ 3000 machine (Corbett Life Sciences) with SYBR Green I detection. The data were analyzed with the comparative CT method (Pfaffl, M.W. (2001) Nuc. Acid Res., 29:2002-2007) that was adapted to quantitate C-raf-1 mRNA relative to GAPDH mRNA. Results are plotted in Figure 5B and an $IC_{50}$ of 17.8 nM was observed, a value that compares favorably with that of the best antisense oligonucleotide (out of 34 tested) called "ISIS 5132" in Monia et al. (Nat Med. (1996) 2:668-75) that had an $IC_{50}$ = 50nM. Notably, LNA13 cannot act through the Rnase H cleavage pathway because it contains a sufficient number of modified nucleotides so that Rnase H activity is inhibited (Kurreck et al. (2002) Nuc. Acids Res., 30:1911-8). Thus LNA13's $IC_{50}$ value of 17.8 nM does not arise from the Rnase H cleavage pathway.

The C-raf-1 3'UTR and sequences past the poly(A) site were subcloned into a Renilla reporter to make the pRL-wtC-raf-1 plasmid. This allowed for the direct comparison of the $IC_{50}$ values with other Renilla reporter plasmids as discussed hereinabove. As shown in Figure 6A, co-transfection of pRL-wtC-raf-1 with LNA13 gave an $IC_{50}$ value of 7.98 nM which is similar to the $IC_{50}$ value seen with the endogenous C-raf-1 gene. Some differences may be expected as the pRL-wtC-raf-1 plasmid produces a chimeric mRNA that may behave differently than the endogenous gene.

To determine the intrinsic inhibitory activity of LNA13, a single LNA13 binding site was inserted into pRL-SV40 and inhibition was tested as described above. An $IC_{50}$ value of ~2 nM was determined. Thus, the inhibitory activity of a single U1 adaptor, in this case LNA13, varies when tested against its endogenous target gene, a reporter plasmid with the natural 3'UTR of the target gene (Figure 6A), and a reporter plasmid with the isolated binding site (Figure 6B). Without being bound by theory, the differences in activity may be due to accessibility. Accessibility factors includes (1) folding of the pre-mRNA sequence, (2) binding of transacting factors, and (3) the rate of 3' end processing of the pre-mRNA.

## EXAMPLE IV

The U1 domain contributes to U1 adaptor activity by its affinity to U1 snRNA and more broadly to the U1 snRNP complex. Although the U1 domain sequence is fixed (unless variant U1 snRNAs are targeted), the U1 domain sequence can be lengthened and its composition can be changed. To this end, replacing the LNA-DNA mixmer design with 100% 2'-O-methyl resulted in only a small decrease in activity (Figure 7). Notably, 2'-O-methyl nucleotides are easier to use during synthesis and have a lower cost as compared to LNA-DNA mixmers. Additionally, having a uniform U1 domain composition simplifies adaptor design as the focus is then on optimizing the annealing domain. Having 100% 2'-O-methyl also reduces self annealing problems as compared to having an LNA-DNA mixmer design or other mixmer combinations. The mixmer annealing domain of LNA6 was also replaced with a matching sequence comprising 100% 2'-O-methyl. However, the adaptor comprising only 2'O-methyl completely lost activity. Extension of the annealing domain (Ome-5 U1 adaptor) failed to restore activity indicating that the presence of only 2'O-methyl in the annealing domain could not simply be compensated for by a longer annealing domain.

The U1 adaptor design has the advantages that inhibition does not require enzymatic activity. Thus, a variety of modified bases may be incorporated into its design. Phosphorothioate (PS) bonds are typically incorporated into antisense molecules to improve their stability when delivered into cells. To test whether PS bonds would affect activity, the activity of two matched adaptors LNA17 and LNA21 that differ only in that LNA21 has PS bonds were compared. As can be seen in Figure 8, these two matched adaptors had similar activities, thereby indicating that PS bonds did not effect activity.

To test whether the U1 domain can be moved to the other end of the U1 adaptor, a set of matching adaptors where the U1 domain was placed at either the 5' or 3' of the annealing domain was synthesized. The activity of these two adaptors was found to be comparable, thereby indicating U1 snRNP access to the U1 domain does not depend on its position relative to the annealing domain. A U1 adaptor comprising two U1 domains on both sides (i.e., a multivalent adaptor) was also synthesized. However, no significant change in activity was found as compared to the monovalent adaptors. This suggests that U1 snRNP binding is not the limiting factor for inhibitory activity *in vivo.* Linker bases were also inserted between the annealing and U1 domains. Notably, no loss or improvement in U1 adaptor activity was found.

## EXAMPLE V

A series of adaptors were synthesized and tested where the U1 domain was held constant and the length and composition of the annealing domain were varied. As seen in Figure 9, shortening the annealing domain can lead to a reduction in adaptor activity. Furthermore, reducing the basepairing potential by substituting DNA bases for LNAs can also reduce activity.

The U1 domain length of the U1 adaptors described hereinabove has been limited to 10 nucleotides, mostly because its natural consensus binding site (i.e., the 5'ss) is 9 to 10 nucleotides long. The 5'-most nucleotide of U1 snRNA is an A and is not thought to play a role in 5'ss binding. However, the effect of this nucleotide on U1 adaptor activity was tested. Matching adaptors that differ only by 1 nucleotide in the U1 domain were compared. As shown in Figure 10, the 1 nucleotide-extended U1 domain gives a significant increase in inhibitory activity

Based on the above results with the additional nucleotide, a matched series of adaptors (the LNA17 series) that incrementally vary the U1 domain length from 7 to 13 nucleotides was tested. U1 adaptor activity was found to steadily increase from no activity (7 nucleotide U1 domain) to high activity (13 nucleotide U1 domain). Given the adaptors in Figures 9 and 10 have different annealing domains, it can be concluded that improvement by longer U1 domains does not depend on the annealing domain. Based on the known structure of U1 snRNA, the higher activity may be because the 12th and 13th nucleotides of the U1 domain insert themselves into stem 1A of U1 snRNA. Stem 1A is highly conserved in U1 snRNAs from yeast to humans, suggesting they are functionally important. Further extensions of the U1 domain will eventually disrupt U1 snRNP conformation, such as disrupting binding of U1-70K to stem loop 1. Thus, further extensions of the adaptors will eventually lead to inactive U1 adaptor activity because of disruption of U1 snRNP inhibitory activity.

## EXAMPLE VI

The U1 adaptors were tested in a variety of cell types. It has been previously shown that U1 in-based gene silencing is active in a broad variety of vertebrate cell lines and primary cells (Fortes et al. (2003) Proc. Natl. Acad. Sci., 100: 8264-8269). To test U1 adaptors, the LNA17-11 adaptor was transfected into the cell lines shown in Figure 12. U1 adaptor activity was found in all cases, though there was some variance in the amount of inhibition.

## EXAMPLE VII

As discussed hereinabove, U1 adaptors and siRNA utilize distinct mechanisms that occur in different compartments of the cell (nucleus versus cytoplasm). To determine whether their combined usage to silence a single gene would give enhanced inhibition, the pRL-LNA6 Renilla reporter plasmid was targeted with 1 nM anti-Renilla siRNA (RL-siRNA) from Ambion/ABI (catalog 4630; Austin, TX) and 30 nM LNA6 adaptor. Control siRNA (Ctr-siRNA) from Ambion/ABI (catalog 4611G) and the LNA7 control adaptor were used as controls. As shown in Figure 13A, the co-transfection of RL-siRNA with LNA6 gave markedly enhanced inhibition of Renilla expression when compared to use of the control oligos.

To rule out that these results depend on the type of adaptor and reporter, an anti-GAPDH U1 adaptor (LNA12) was used in place of ULNA6 and the reporter plasmid having an annealing site for LNA12 because it contains the 3'UTR of the human GAPDH mRNA (Figure 13B). More specifically, pRL-GAPDH is a Renilla reporter with the 3'UTR and poly (A) signal sequences derived from the human GAPDH mRNA (GenBank Accession No. NM_002046) plus 200 basepairs past the GAPDH poly(A) signal. The LNA12 adaptor is targeting 1231-1245, using Gen Bank Accession No. NM_002046 coordinates.

As shown in Figure 13C, enhanced activity was observed when 15 nM LNA12 and 1 nM RL-siRNA were used together. Although the siRNAs used in these experiments are more active in silencing than the U1 adaptors, it should be noted that the siRNAs were optimized over the course of years to produce such highly active siRNAs to GAPDH and to Renilla reporter plasmids.

In view of the above data, the combination of U1 adaptors with more traditional antisense-based methods that employ RNase H activity were tested. In this experiment, the pRL-wtC-raf reporter plasmid was targeted. The LNA25-H/U1 oligo combines into one molecule both adaptor and Rnase H activities by designing the annealing domain to have an uninterrupted stretch of at least seven DNA bases (in this case 10 bases) as seen for LNA25-H/U1. Such a "7nt DNA design" was shown by Grünweller et al. (Nuc. Acids Res. (2003) 31:3185-93) to be sufficient for Rnase H activity, although longer stretches are more active. LNA25-mtH/U1 matches LNA25-H/U1 but has the stretch of DNAs interrupted and so should not have Rnase H activity. In like manner, LNA25-H/mtU1 matches LNA25-H/U1 but has a 2 nucleotide mutation in the U1 domain (Figure 14A). This design is such that LNA25-H/mtU1 has only Rnase H activity, LNA25-mtH/U1 has only U1 adaptor activity, and LNA25-H/U1 will have both activities. As can be seen in Figure 14B, the LNA25-H/U1 has higher activity indicating both silencing methods can give enhanced activity when used together. Notably, DNA stretches that are longer than 10 nucleotides can be used, however the potential for self annealing of oligos with longer DNA stretches needs to be considered.

## EXAMPLE VIII

To determine whether the LNA25 series of oligonucleotides can inhibit expression of the endogenous C-raf-1 gene, HeLa cells were transfected with LNA25-mtH/U1 and Western blotting combined with Q-PCR was performed. The results in Figure 15 show specific silencing of C-raf-1 both at the protein and mRNA levels. It has been reported that silencing of C-raf-1 leads to induction of cleavage of the PARP protein as part of induction of apoptosis (Lau et al. (1998) Oncogene 16:1899-902). Re-probing the Western blot in Figure 15A with anti-PARP antibody demonstrated that the U1 adaptors induce PARP cleavage indicative of C-raf-1 silencing.

As described hereinabove, the combinatorial use of adaptors and Rnase H gave enhanced silencing of a reporter plasmid. To determine whether enhanced activity could be extended to silencing of the endogenous C-raf-1 gene, the above transfections in Example VII were repeated, but now the levels of the endogenous C-raf-1 protein and mRNA were measured. The results in Figure 16 show enhanced silencing of C-raf-1 both at the protein and mRNA levels.

While certain of the preferred embodiments of the present invention have been described and specifically exemplified above, it is not intended that the invention be limited to such embodiments.

## Claims

1. A nucleic acid molecule for inhibiting the expression of a gene of interest, wherein said nucleic acid molecule comprises an annealing domain operably linked to at least one effector domain, wherein said annealing domain hybridizes to the pre-mRNA of said gene of interest, and wherein said effector domain hybridizes to the U1 snRNA of U1 snRNP.

2. The nucleic acid molecule of claim 1, wherein said annealing domain is about 10 to about 30 nucleotides in length.

3. The nucleic acid molecule of claim 1, wherein said effector domain is about 8 to about 20 nucleotides in length.

4. The nucleic acid molecule of claim 1, wherein said effector domain and annealing domain are linked by a bond or by a linker domain of about 1 to about 10 nucleotides.

5. The nucleic acid molecule of claim 1, wherein said effector domain comprises the sequence:5'-CAGGUAAGUA-3' (SEQ ID NO: 1), the sequence 5'-CAGGUAAGUAU-3' (SEQ ID NO: 32); or the sequence 5'-GCCAGGUAAGUAU-3' (SEQ ID NO: 33).

6. The nucleic acid molecule of claim 1, wherein said nucleic acid molecule comprises at least one nucleotide analog.

7. The nucleic acid molecule of claim 6, wherein said nucleotide analog is selected from the group consisting of locked nucleic acids and 2'-O-methylnucleotides.

8. The nucleic acid molecule of claim 1, wherein said annealing domain hybridizes with a target sequence in the 3' terminal exon of the gene of interest.

9. The nucleic acid molecule of claim 1, wherein the effector domain is operably linked to the 3' end of the annealing domain, the 5' end of the annealing domain, or both the 5' and 3' end of the annealing domain.

10. The nucleic acid molecule of claim 1, wherein said annealing domain comprises a stretch of at least seven deoxyribonucleotides.

11. An in vitro method of inhibiting the expression of a gene of interest comprising delivering to a cell at least one nucleic acid molecule as recited in claim 1.

12. The method of claim 11, wherein at least two nucleic acid molecules as recited in claim 1 are delivered and wherein the annealing domains of said nucleic acid molecules hybridize with different target sequences in said gene of interest.

13. A composition comprising at least one nucleic acid molecule of claim 1 and at least one pharmaceutically acceptable carrier.

14. The composition of claim 13, wherein said composition further comprises at least one siRNA or antisense oligonucleotide directed against said gene of interest.

15. A vector encoding the nucleic acid molecule of claim 1.

16. The vector of claim 15, wherein said vector is a viral vector or a plasmid.

## Patentansprüche

1. Nucleinsäuremolekül zur Hemmung der Expression eines interessierenden Gens, wobei das Nucleinsäuremolekül eine Annealing-Domäne, die funktional mit mindestens einer Effektordomäne verknüpft ist, umfasst, wobei die Annealing-Domäne mit der prä-mRNA des interessierenden Gens hybridisiert und wobei die Effektordomäne mit der U1-snRNA von U1-snRNP hybridisiert.

2. Nucleinsäuremolekül nach Anspruch 1, wobei die Annealing-Domäne eine Länge von etwa 10 bis etwa 30 Nucleotiden aufweist.

3. Nucleinsäuremolekül nach Anspruch 1, wobei die Effektordomäne eine Länge von etwa 8 bis etwa 20 Nucleotiden aufweist.

4. Nucleinsäuremolekül nach Anspruch 1, wobei die Effektordomäne und die Annealing-Domäne durch eine Bindung oder durch eine Linkerdomäne von etwa 1 bis etwa 10 Nucleotiden verknüpft sind.

5. Nucleinsäuremolekül nach Anspruch 1, wobei die Effektordomäne
   die Sequenz 5'-CAGGUAAGUA-3' (SEQ ID NO: 1),
   die Sequenz 5'-CAGGUAAGUAU-3' (SEQ ID NO: 32) oder
   die Sequenz 5'-GCCAGGUAAGUAU-3' (SEQ ID NO: 33) umfasst.

6. Nucleinsäuremolekül nach Anspruch 1, wobei das Nucleinsäuremolekül mindestens ein Nucleotidanalogon umfasst.

7. Nucleinsäuremolekül nach Anspruch 6, wobei das Nucleotidanalogon aus der Gruppe von Locked-Nucleinsäuren und 2'-O-Methylnucleotiden ausgewählt ist.

8. Nucleinsäuremolekül nach Anspruch 1, wobei die Annealing-Domäne mit einer Zielsequenz im 3'-terminalen Exon des interessierenden Gens hybridisiert.

9. Nucleinsäuremolekül nach Anspruch 1, wobei die Effektordomäne funktional mit dem 3'-Ende der Annealing-Domäne, dem 5'-Ende der Annealing-Domäne oder sowohl dem 5'- als auch dem 3'-Ende der Annealing-Domäne verknüpft ist.

10. Nucleinsäuremolekül nach Anspruch 1, wobei die Annealing-Domäne eine Strecke von mindestens sieben Desoxyribonucleotiden umfasst.

11. In-vitro-Verfahren zur Hemmung der Expression eines interessierenden Gens, umfassend das Zuführen von mindestens einem Nucleinsäuremolekül gemäß Anspruch 1 in eine Zelle.

12. Verfahren nach Anspruch 11, wobei mindestens zwei Nucleinsäuremoleküle gemäß Anspruch 1 zugeführt werden und wobei die Annealing-Domänen der Nucleinsäuremoleküle mit verschiedenen Zielsequenzen in dem interessierenden Gen hybridisieren.

13. Zusammensetzung, die mindestens ein Nucleinsäuremolekül nach Anspruch 1 und mindestens einen pharmazeutisch akzeptablen Träger umfasst.

14. Zusammensetzung nach Anspruch 13, wobei die Zusammensetzung ferner mindestens eine siRNA oder ein Antisense-Oligonucleotid, die auf das interessierende Gen gerichtet sind, umfasst.

15. Vektor mit Codierung für das Nucleinsäuremolekül nach Anspruch 1.

16. Vektor nach Anspruch 15, wobei der Vektor ein viraler Vektor oder ein Plasmid ist.

## Revendications

1. Molécule d'acide nucléique pour inhiber l'expression d'un gène d'intérêt, dans laquelle ladite molécule d'acide nucléique comprend un domaine d'hybridation lié de façon opérationnelle à au moins un domaine effecteur, dans laquelle ledit domaine d'hybridation s'hybride au pré-ARNm dudit gène d'intérêt, et dans laquelle ledit domaine effecteur s'hybride au petit ARN nucléaire U1 de la RNPnp U1.

2. Molécule d'acide nucléique selon la revendication 1, dans laquelle ledit domaine d'hybridation a une longueur d'environ 10 à environ 30 nucléotides.

3. Molécule d'acide nucléique selon la revendication 1, dans laquelle ledit domaine effecteur a une longueur d'environ 8 à environ 20 nucléotides.

4. Molécule d'acide nucléique selon la revendication 1, dans laquelle lesdits domaine effecteur et domaine d'hybridation sont liés par une liaison ou par un domaine de liaison d'environ 1 à environ 10 nucléotides.

5. Molécule d'acide nucléique selon la revendication 1, dans laquelle ledit domaine effecteur comprend la séquence : 5'-CAGGUAAGUA-3' (SEQ ID NO : 1), la séquence 5'-CAGGUAAGUAU-3' (SEQ ID NO : 32) ; ou la séquence 5'-GCCAGGUAAGUAU-3' (SEQ ID NO : 33).

6. Molécule d'acide nucléique selon la revendication 1, dans laquelle ladite molécule d'acide nucléique comprend au moins un analogue de nucléotide.

7. Molécule d'acide nucléique selon la revendication 6, dans laquelle ledit analogue de nucléotide est choisi dans le groupe constitué par les acides nucléiques verrouillés et les 2'-O-méthylnucléotides.

8. Molécule d'acide nucléique selon la revendication 1, dans laquelle ledit domaine d'hybridation s'hybride avec une séquence cible dans l'exon terminal en 3' du gène d'intérêt.

9. Molécule d'acide nucléique selon la revendication 1, dans laquelle le domaine effecteur est lié de façon opérationnelle à l'extrémité 3' du domaine d'hybridation, l'extrémité 5' du domaine d'hybridation, ou à la fois les extrémités 5' et 3' du domaine d'hybridation.

10. Molécule d'acide nucléique selon la revendication 1, dans laquelle ledit domaine d'hybridation comprend un allongement d'au moins sept désoxyribonucléotides.

11. Procédé *in vitro* d'inhibition de l'expression d'un gène d'intérêt comprenant la fourniture à une cellule d'au moins une molécule d'acide nucléique selon la revendication 1.

12. Procédé selon la revendication 11, dans lequel au moins deux molécules d'acide nucléique selon la revendication 1 sont fournies et dans lequel les domaines d'hybridation desdites molécules d'acide nucléique s'hybrident avec différentes séquences cibles dans ledit gène d'intérêt.

13. Composition comprenant au moins une molécule d'acide nucléique selon la revendication 1 et au moins un véhicule pharmaceutiquement acceptable.

14. Composition selon la revendication 13, dans laquelle ladite composition comprend en outre au moins un petit ARNi ou oligonucléotide antisens dirigé contre ledit gène d'intérêt.

15. Vecteur codant la molécule d'acide nucléique selon la revendication 1.

16. Vecteur selon la revendication 15, dans lequel ledit vecteur est un vecteur viral ou un plasmide.

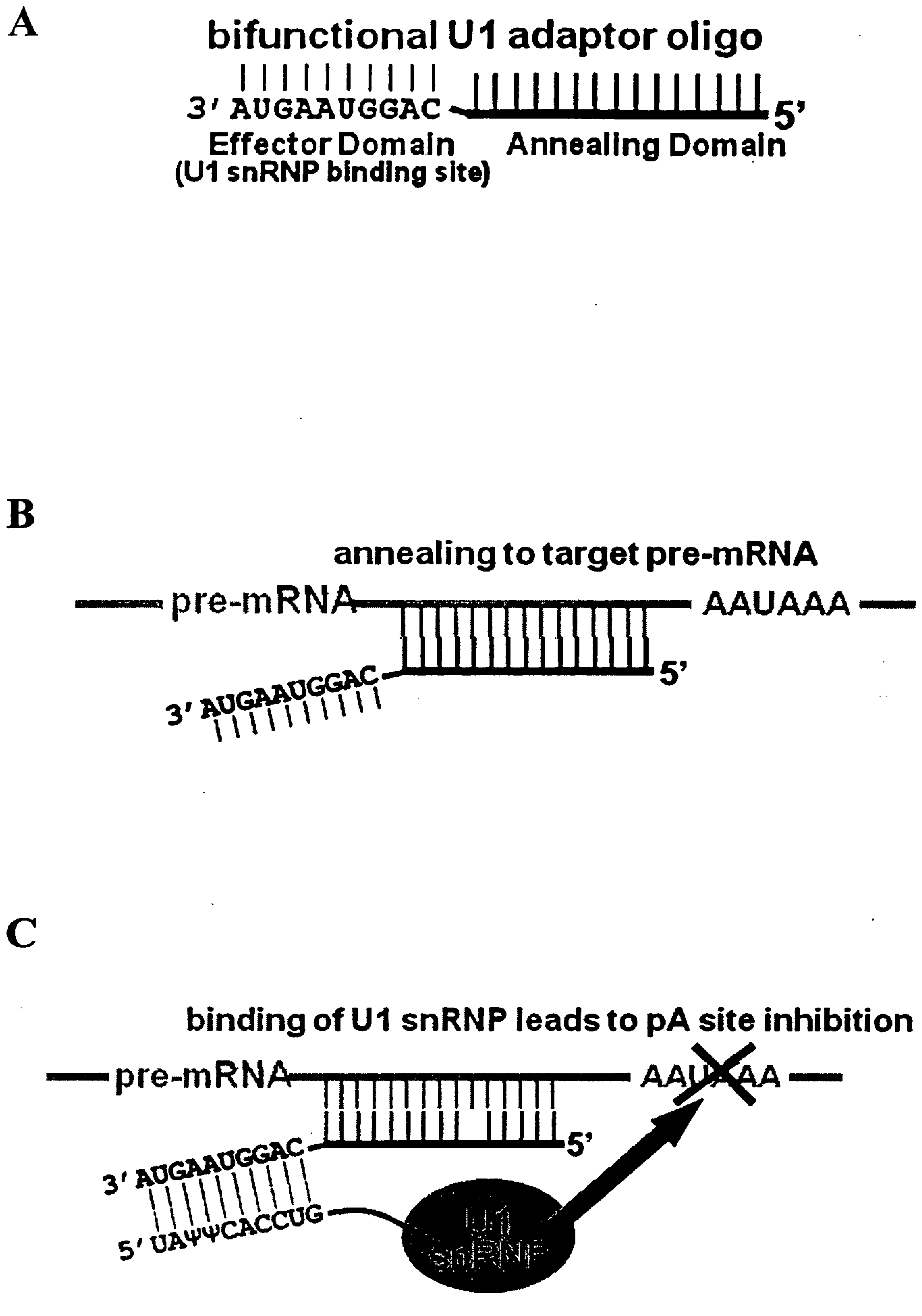
A
bifunctional U1 adaptor oligo
3' AUGAAUGGAC
5'
Effector Domain
(U1 snRNP binding site)
Annealing Domain
B
annealing to target pre-mRNA
pre-mRNA
AAUAAA
3' AUGAAUGGAC
5'
C
binding of U1 snRNP leads to pA site inhibition
pre-mRNA
AAUAAA
3' AUGAAUGGAC
5'
5' UAΨΨCACCUG
U1
snRNP

Figure 1

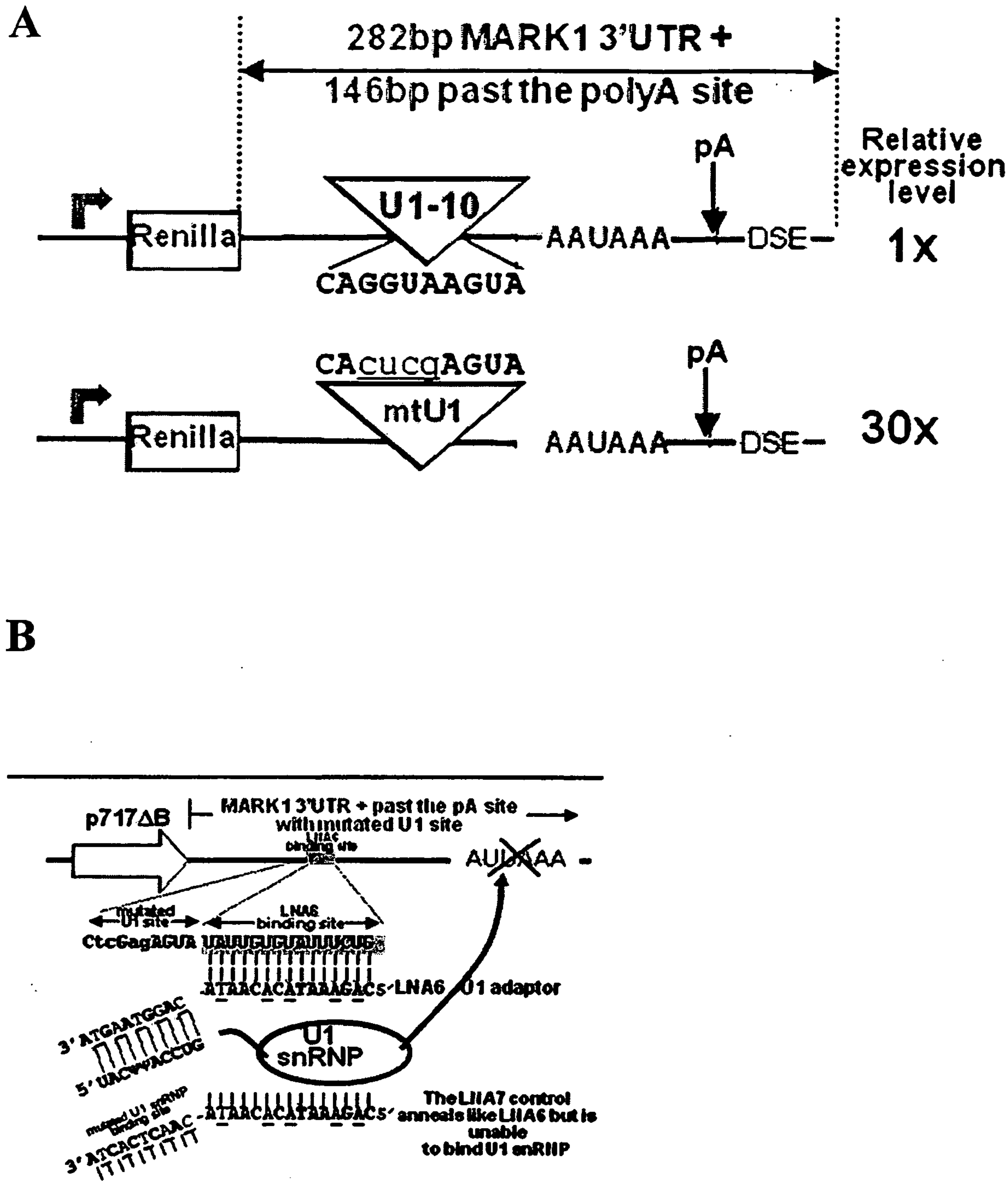
A
282bp MARK1 3'UTR +
146bp past the polyA site
Relative expression level
pA
Renilla
U1-10
AAUAAA
DSE
1x
CAGGUAAGUA
CAcucgAGUA
pA
Renilla
mtU1
AAUAAA
DSE
30x
B
p717ΔB
MARK1 3'UTR + past the pA site with mutated U1 site
AUUAAA
mutated U1 site
LNA6 binding site
CtcGagAGUA
ATAACACATAAAGAC5'-LNA6 U1 adaptor
U1 snRNP
3' ATGAATGGAC
5' UACWWACCUG
ATAACACATAAAGAC5'
3' ATCACTCAAC
The LNA7 control anneals like LNA6 but is unable to bind U1 snRNP

Figure 2

A

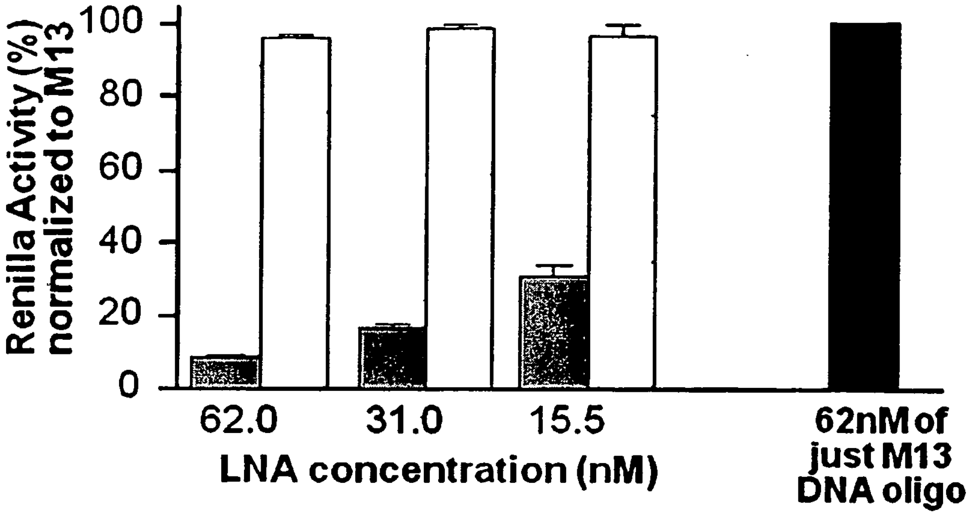
Renilla Activity (%) normalized to M13
100
80
60
40
20
0
62.0
31.0
15.5
LNA concentration (nM)
62nM of just M13 DNA oligo

B

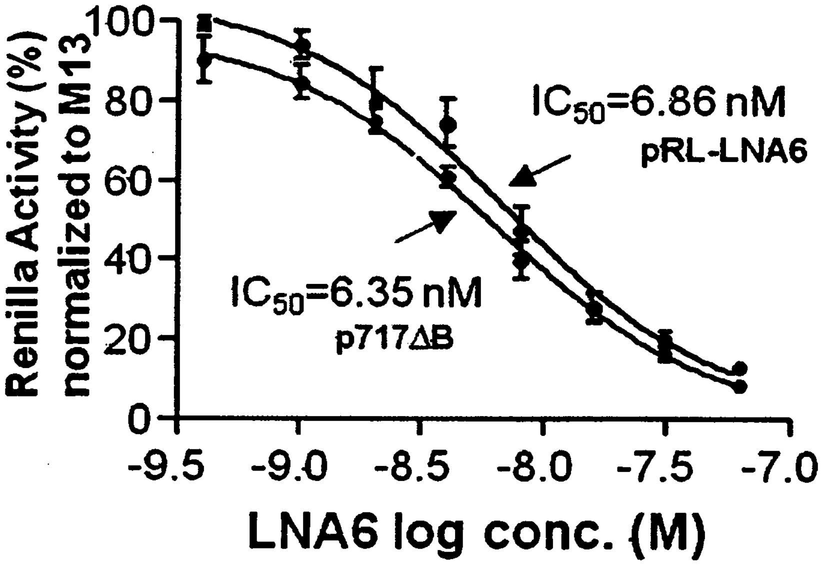
Renilla Activity (%) normalized to M13
100
80
60
40
20
0
-9.5
-9.0
-8.5
-8.0
-7.5
-7.0
LNA6 log conc. (M)
IC50=6.86 nM
pRL-LNA6
IC50=6.35 nM
p717ΔB

C

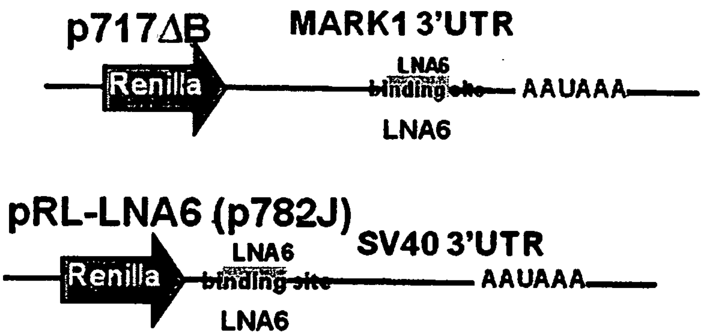
p717ΔB
MARK1 3'UTR
Renilla
LNA6
binding site
AAUAAA
LNA6
pRL-LNA6 (p782J)
SV40 3'UTR
Renilla
LNA6
binding site
AAUAAA
LNA6

Figure 3

A

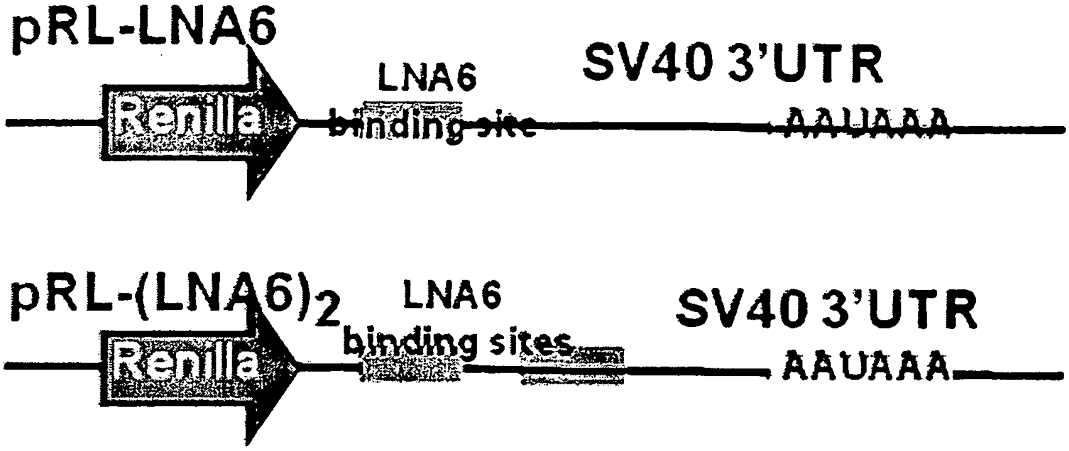
pRL-LNA6
LNA6
binding site
SV40 3'UTR
Renilla
AAUAAA
pRL-(LNA6)2
LNA6
binding sites
SV40 3'UTR
Renilla
AAUAAA

B

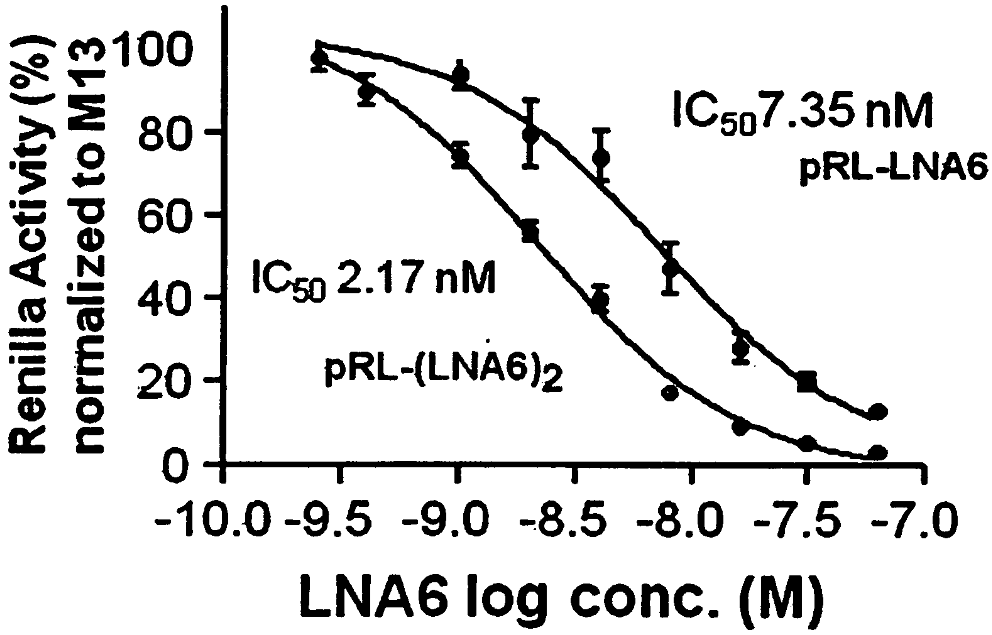
Renilla Activity (%)
normalized to M13
100
80
60
40
20
0
IC50 7.35 nM
pRL-LNA6
IC50 2.17 nM
pRL-(LNA6)2
-10.0 -9.5 -9.0 -8.5 -8.0 -7.5 -7.0
LNA6 log conc. (M)

**Figure 4**

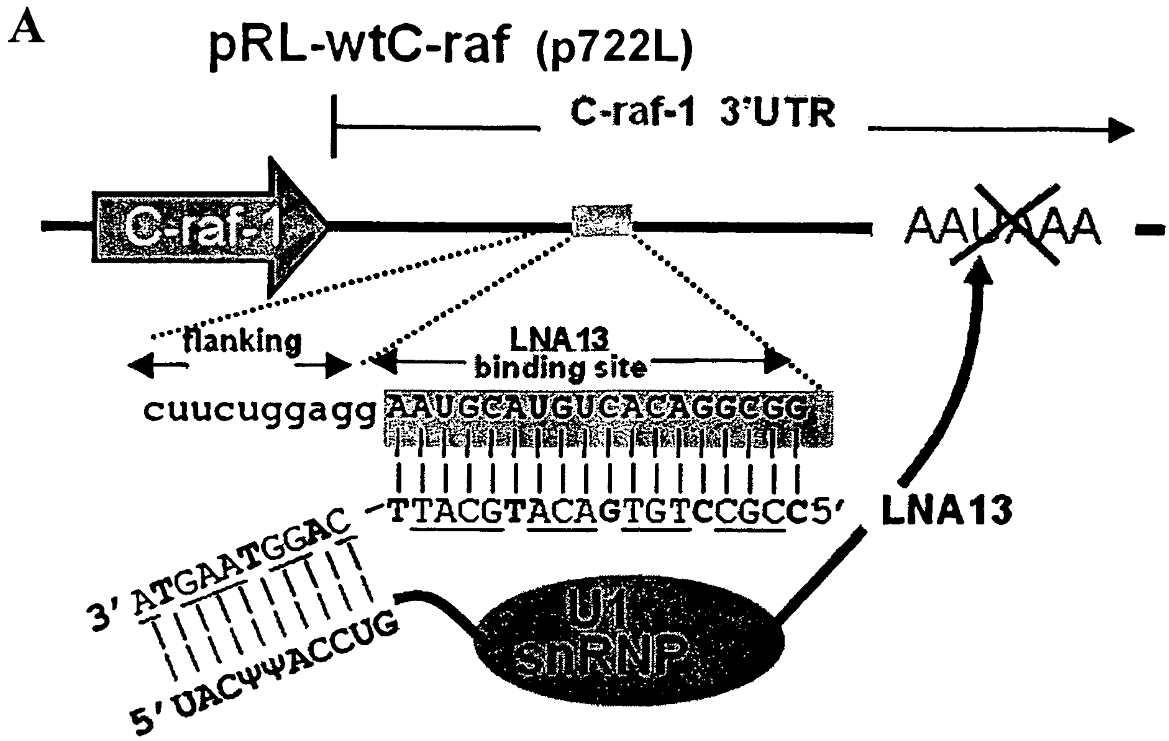
A
pRL-wtC-raf (p722L)
C-raf-1 3'UTR
C-raf-1
AAUAAA
flanking
LNA13 binding site
cuucuggagg AAUGCAUGUCACAGGCGG
TTACGTACAGTGTCCGCC5'
LNA13
3' ATGAATGGAC
5' UACΨΨACCUG
U1 snRNP

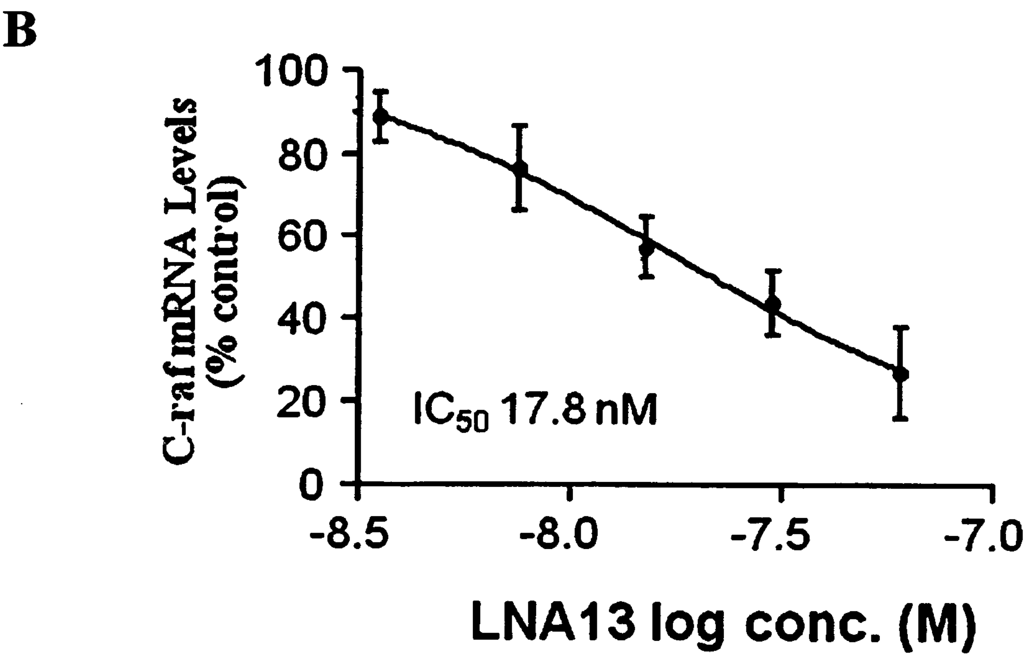
B
C-raf mRNA Levels (% control)
100
80
60
40
20
0
IC50 17.8 nM
-8.5
-8.0
-7.5
-7.0
LNA13 log conc. (M)

Figure 5

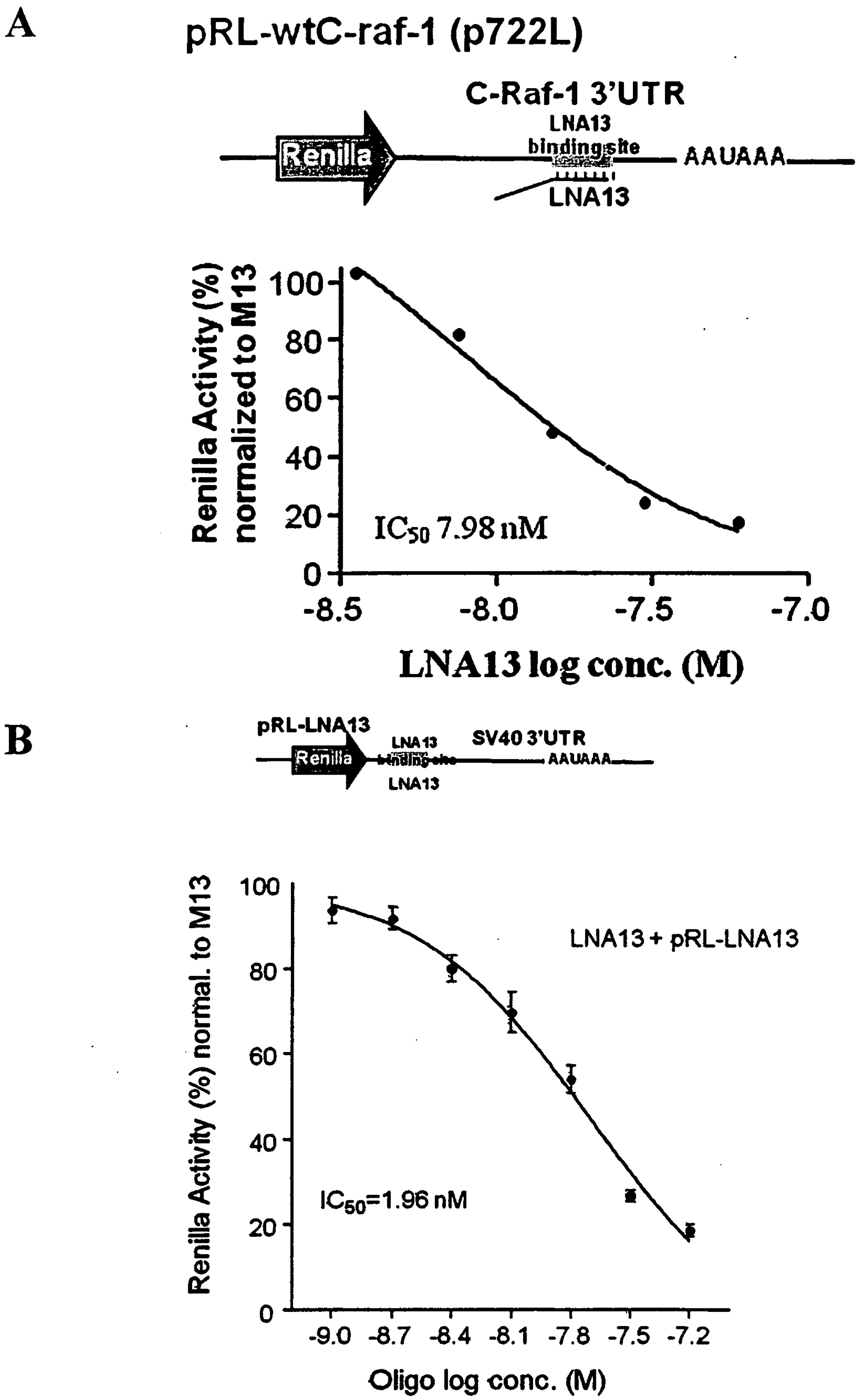
A
pRL-wtC-raf-1 (p722L)
C-Raf-1 3'UTR
LNA13
binding site
Renilla
AAUAAA
LNA13
Renilla Activity (%) normalized to M13
100
80
60
40
20
0
IC50 7.98 nM
-8.5
-8.0
-7.5
-7.0
LNA13 log conc. (M)
B
pRL-LNA13
LNA13
binding site
SV40 3'UTR
Renilla
AAUAAA
LNA13
Renilla Activity (%) normal. to M13
100
80
60
40
20
0
LNA13 + pRL-LNA13
IC50=1.96 nM
-9.0
-8.7
-8.4
-8.1
-7.8
-7.5
-7.2
Oligo log conc. (M)

**Figure 6**

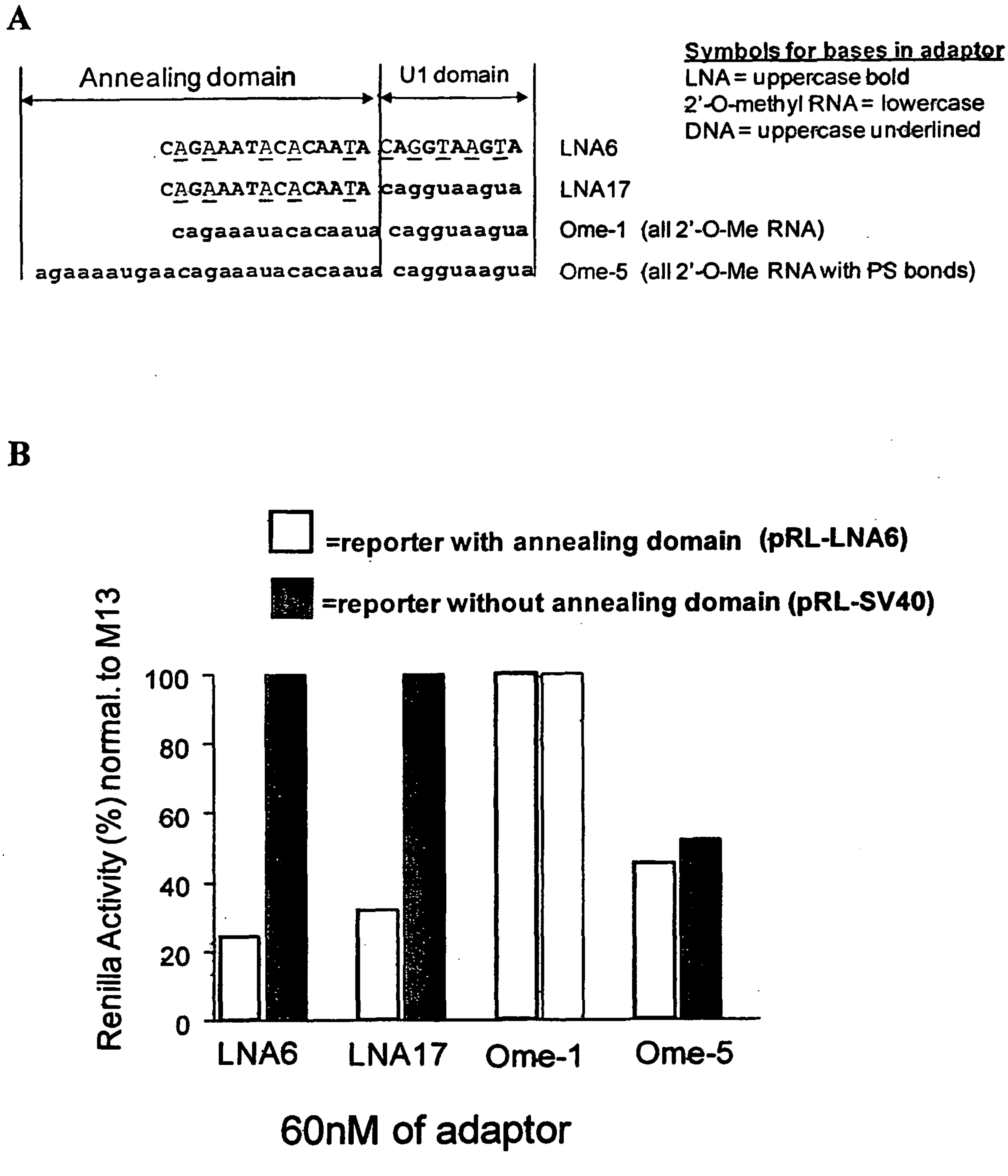
A
Annealing domain
U1 domain
CAGAAATACACAATA CAGGTAAGTA LNA6
CAGAAATACACAATA cagguaagua LNA17
cagaaauacacaaua cagguaagua Ome-1 (all 2'-O-Me RNA)
agaaaaugaacagaaauacacaaua cagguaagua Ome-5 (all 2'-O-Me RNA with PS bonds)
Symbols for bases in adaptor
LNA = uppercase bold
2'-O-methyl RNA = lowercase
DNA = uppercase underlined
B
=reporter with annealing domain (pRL-LNA6)
=reporter without annealing domain (pRL-SV40)
Renilla Activity (%) normal. to M13
100
80
60
40
20
0
LNA6
LNA17
Ome-1
Ome-5
60nM of adaptor

Figure 7

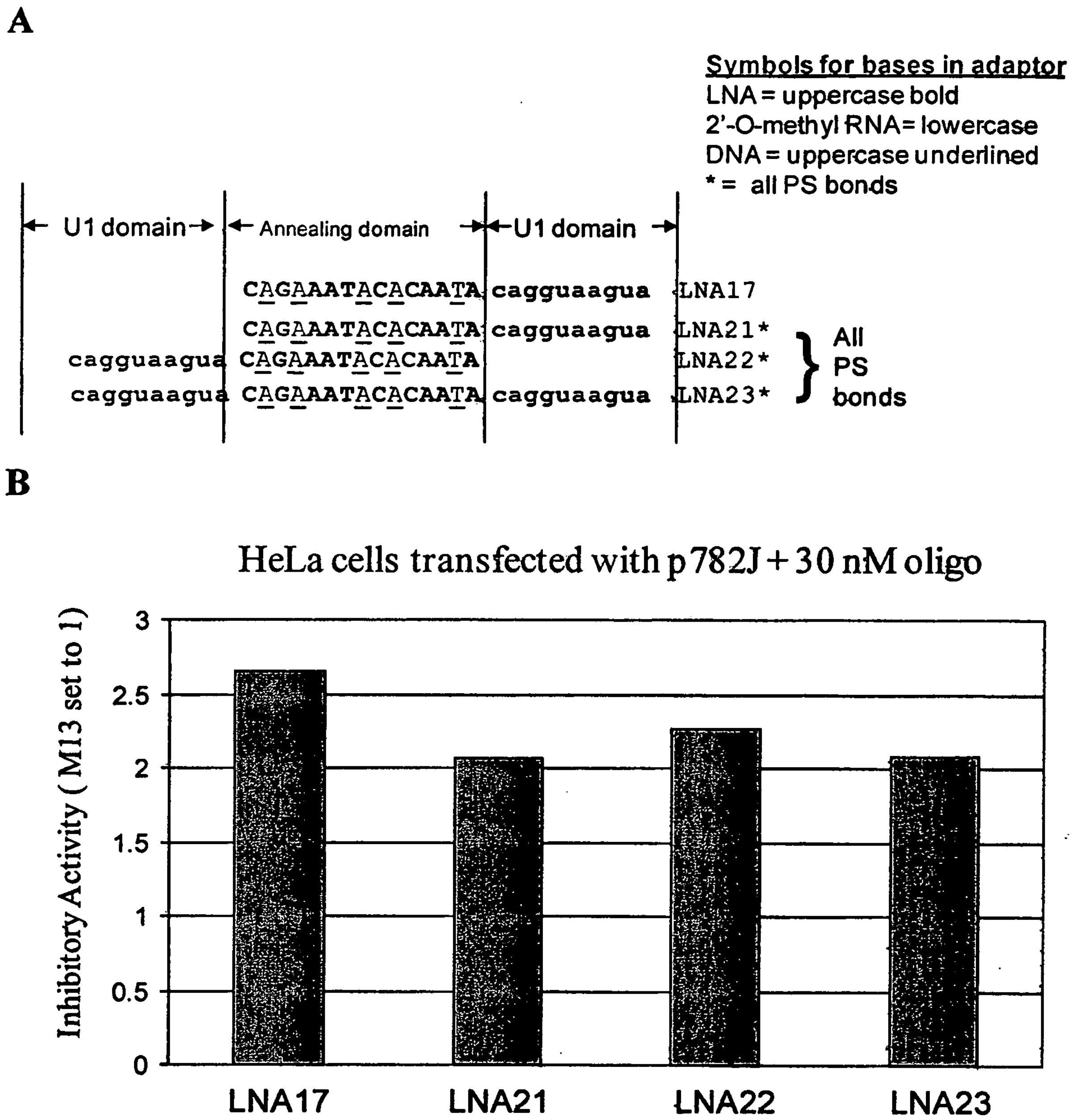
A
Symbols for bases in adaptor
LNA = uppercase bold
2'-O-methyl RNA = lowercase
DNA = uppercase underlined
* = all PS bonds
U1 domain
Annealing domain
U1 domain
CAGAAATACACAATA cagguaagua LNA17
CAGAAATACACAATA cagguaagua LNA21*
cagguaagua CAGAAATACACAATA LNA22*
cagguaagua CAGAAATACACAATA cagguaagua LNA23*
All PS bonds
B
HeLa cells transfected with p782J + 30 nM oligo
Inhibitory Activity (M13 set to 1)
3
2.5
2
1.5
1
0.5
0
LNA17
LNA21
LNA22
LNA23

**Figure 8**

A

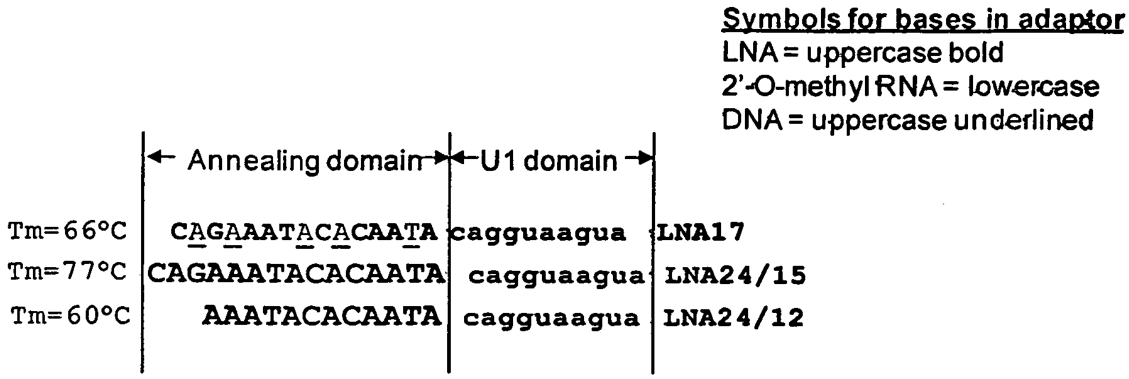
Symbols for bases in adaptor
LNA = uppercase bold
2'-O-methyl RNA = lowercase
DNA = uppercase underlined
Annealing domain
U1 domain
Tm=66°C
CAGAAATACACAATA
cagguaagua
LNA17
Tm=77°C
CAGAAATACACAATA
cagguaagua
LNA24/15
Tm=60°C
AAATACACAATA
cagguaagua
LNA24/12

B

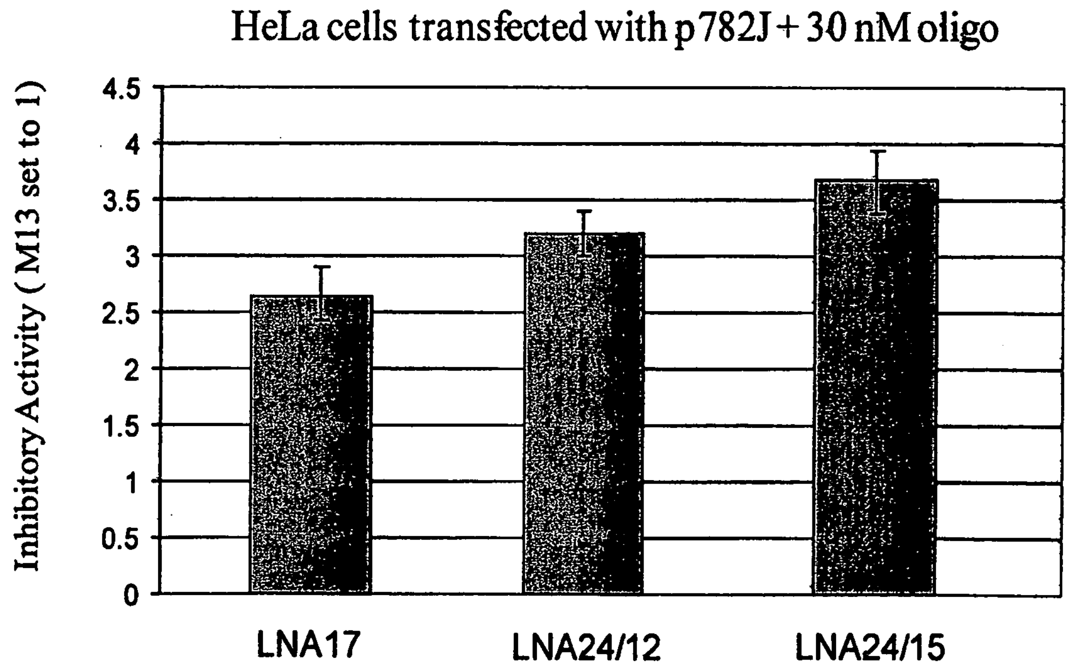
HeLa cells transfected with p782J + 30 nM oligo
Inhibitory Activity ( M13 set to 1)
4.5
4
3.5
3
2.5
2
1.5
1
0.5
0
LNA17
LNA24/12
LNA24/15

**Figure 9**

**Symbols for bases in adaptor**

LNA = uppercase bold
2'-O-methyl RNA = lowercase
DNA = uppercase underlined

pRL-wtC-raf-1 (p722L)

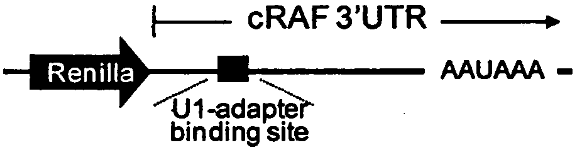


Cotransfection: p722L + 30nM oligo
Fold Change (M13 set to 1)

| Annealing domain | U1 domain | | Avg. | SD |
|---|---|---|---|---|
| CCGCCTGTGACATGCATTT | CAGGTAAGTA | LNA13 | 3.22 | 0.26 |
| CCGCCTGTGACATGCATTT | cagguaaguau | LNA25-mtH/U1 | 8.57 | 1.29 |

**Figure 10**

**A**

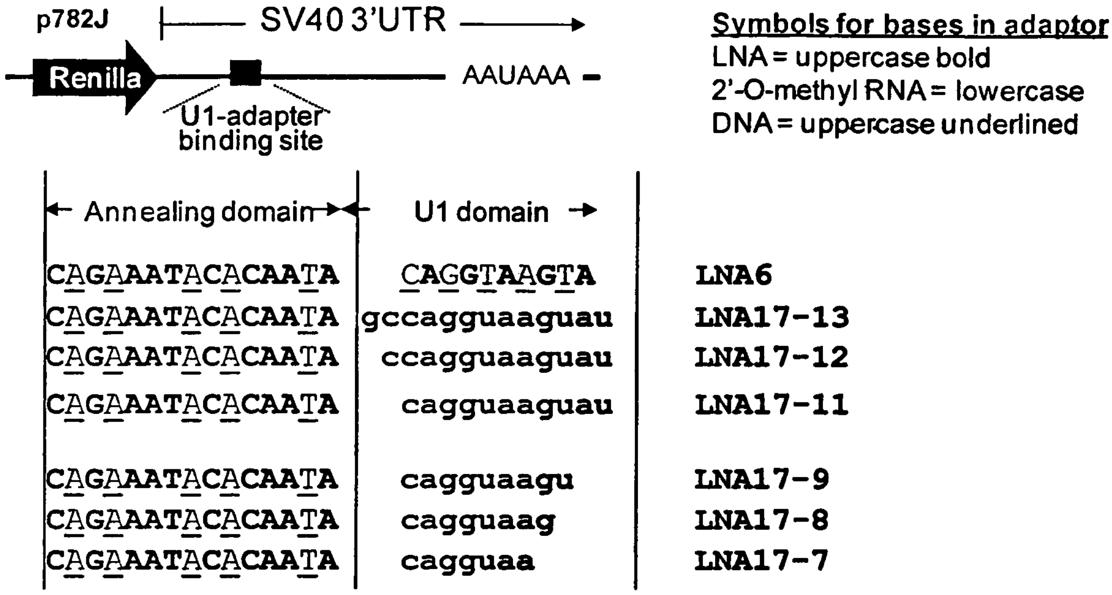
p782J
SV40 3'UTR
Renilla
AAUAAA
U1-adapter binding site
Symbols for bases in adaptor
LNA = uppercase bold
2'-O-methyl RNA = lowercase
DNA = uppercase underlined
Annealing domain
U1 domain
CAGAAATACACAATA CAGGTAAGTA LNA6
CAGAAATACACAATA gccagguaaguau LNA17-13
CAGAAATACACAATA ccagguaaguau LNA17-12
CAGAAATACACAATA cagguaaguau LNA17-11
CAGAAATACACAATA cagguaagu LNA17-9
CAGAAATACACAATA cagguaag LNA17-8
CAGAAATACACAATA cagguaa LNA17-7

**B**

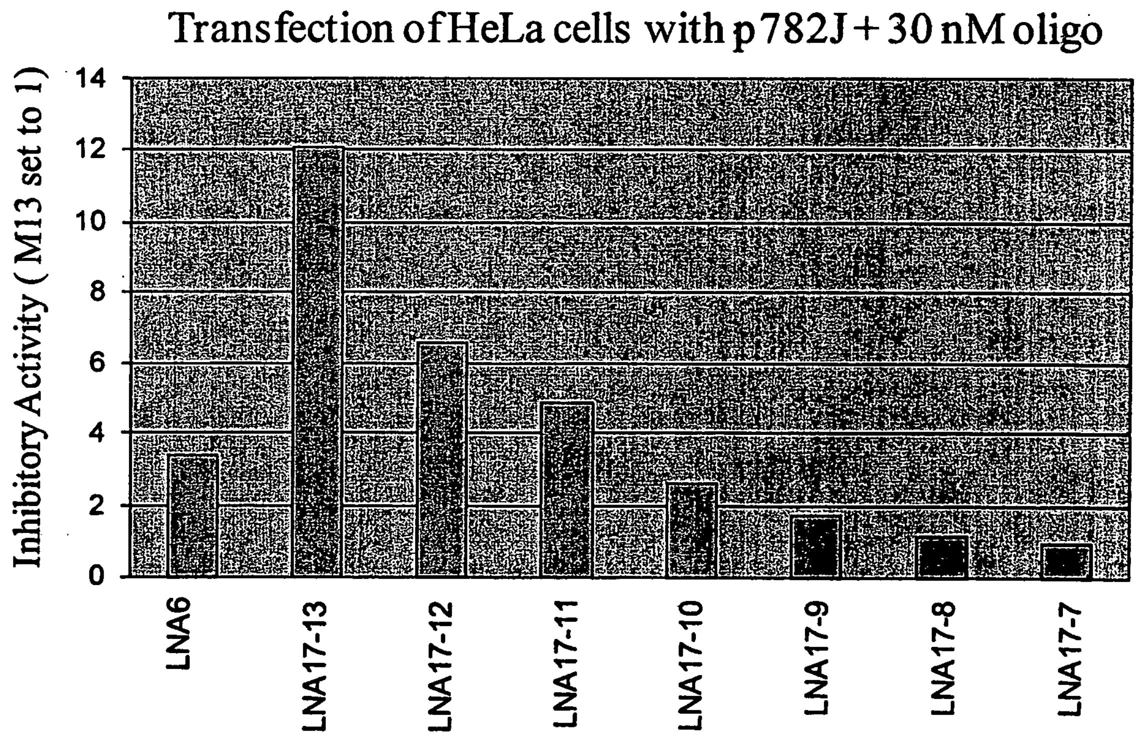
Transfection of HeLa cells with p782J + 30 nM oligo
Inhibitory Activity ( M13 set to 1)
14
12
10
8
6
4
2
0
LNA6
LNA17-13
LNA17-12
LNA17-11
LNA17-10
LNA17-9
LNA17-8
LNA17-7

**Figure 11**

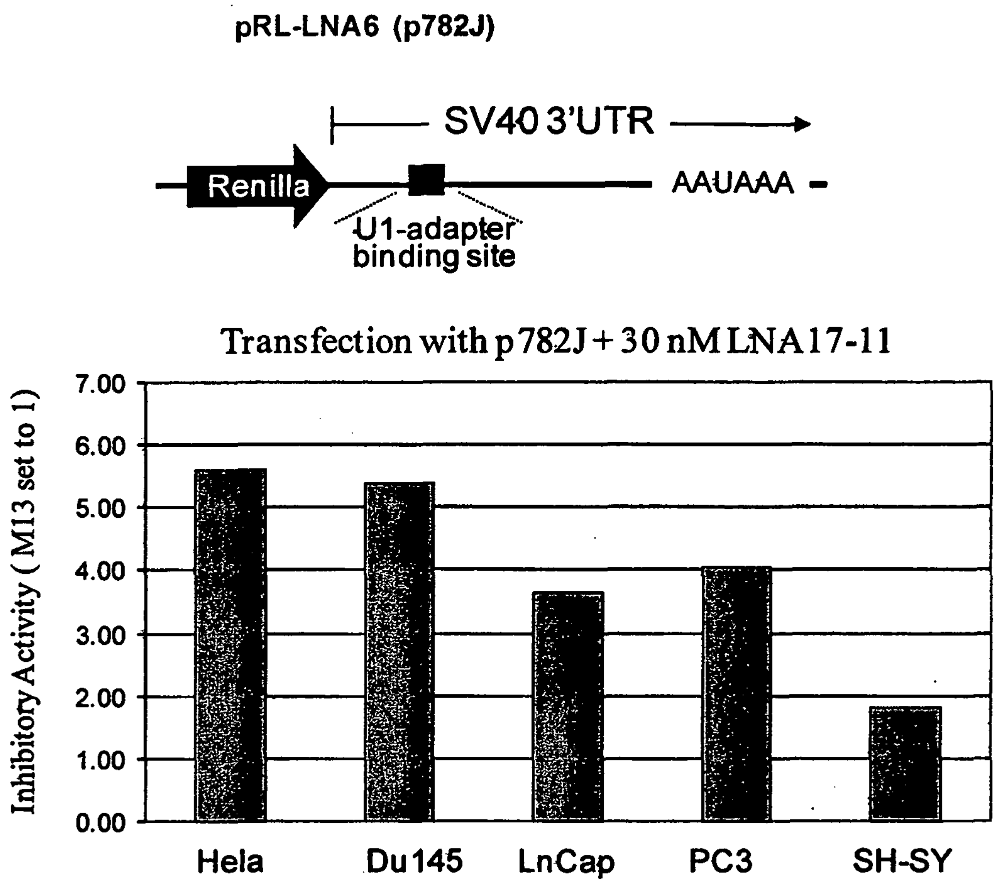

pRL-LNA6 (p782J)
SV40 3'UTR
Renilla
AAUAAA
U1-adapter binding site
Transfection with p782J + 30 nM LNA17-11
Inhibitory Activity ( M13 set to 1)
7.00
6.00
5.00
4.00
3.00
2.00
1.00
0.00
Hela
Du145
LnCap
PC3
SH-SY


**Figure 12**

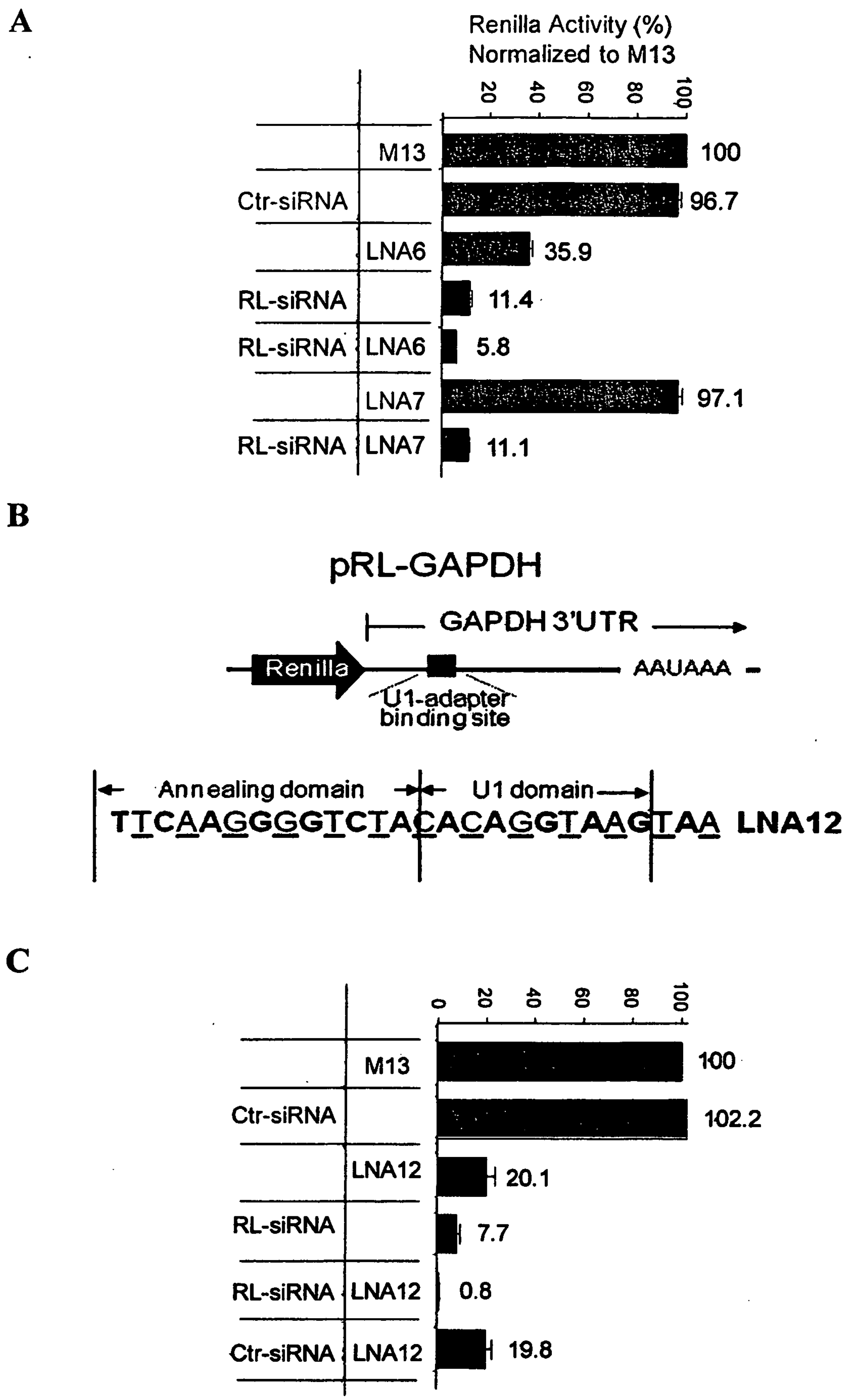
A
Renilla Activity (%)
Normalized to M13
20
40
60
80
100
M13
100
Ctr-siRNA
96.7
LNA6
35.9
RL-siRNA
11.4
RL-siRNA
LNA6
5.8
LNA7
97.1
RL-siRNA
LNA7
11.1
B
pRL-GAPDH
GAPDH 3'UTR
Renilla
AAUAAA
U1-adapter binding site
Annealing domain
U1 domain
TTCAAGGGGTCTACACAGGTAAGTAA LNA12
C
0
20
40
60
80
100
M13
100
Ctr-siRNA
102.2
LNA12
20.1
RL-siRNA
7.7
RL-siRNA
LNA12
0.8
Ctr-siRNA
LNA12
19.8

**Figure 13**

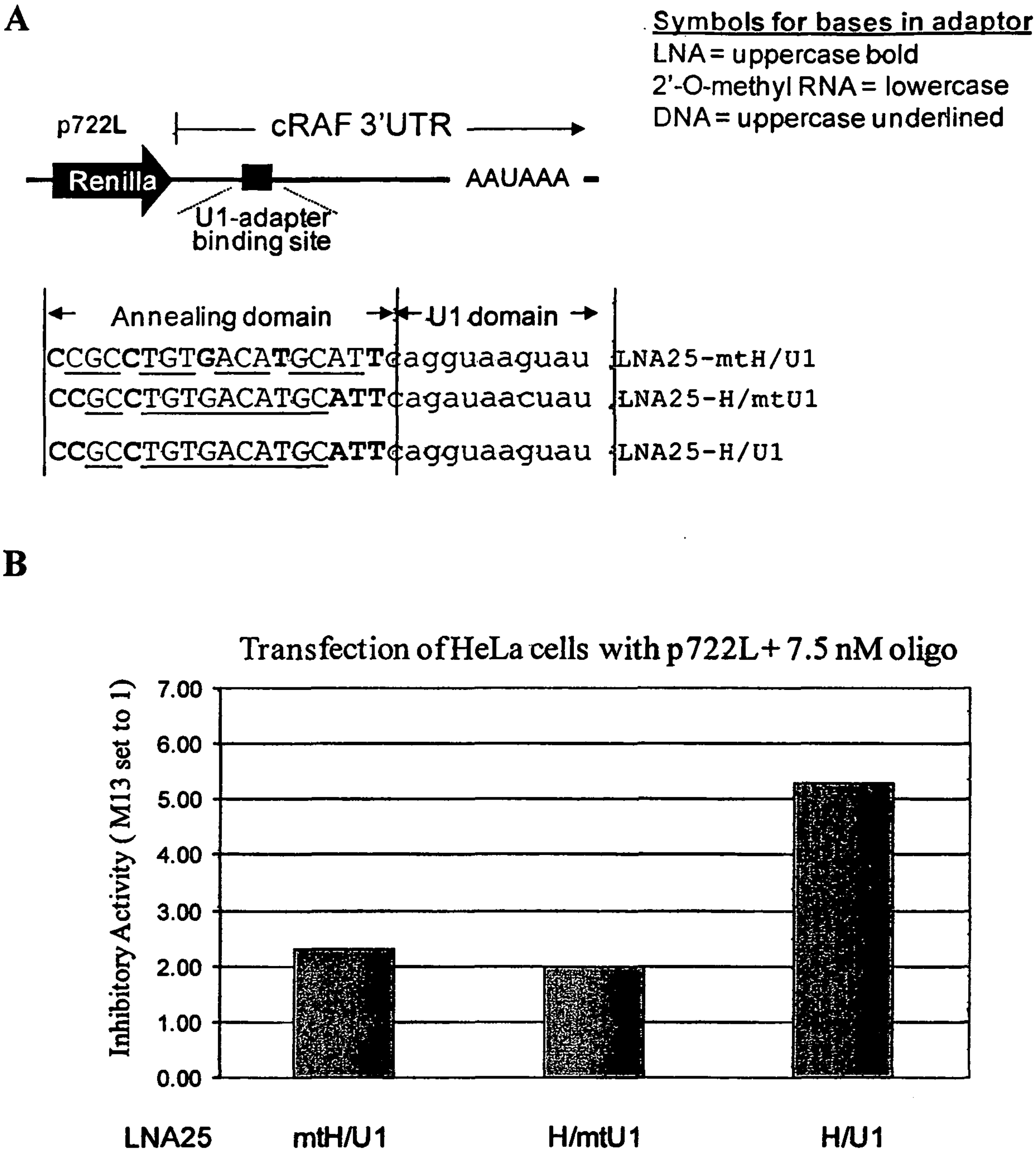
A
Symbols for bases in adaptor
LNA = uppercase bold
2'-O-methyl RNA = lowercase
DNA = uppercase underlined
p722L
cRAF 3'UTR
Renilla
AAUAAA
U1-adapter binding site
Annealing domain
U1 domain
CCGCCTGTGACATGCATTcagguaaguau LNA25-mtH/U1
CCGCCTGTGACATGCATTcagauaacuau LNA25-H/mtU1
CCGCCTGTGACATGCATTcagguaaguau LNA25-H/U1
B
Transfection of HeLa cells with p722L + 7.5 nM oligo
Inhibitory Activity (M13 set to 1)
7.00
6.00
5.00
4.00
3.00
2.00
1.00
0.00
LNA25
mtH/U1
H/mtU1
H/U1

**Figure 14**

A

B

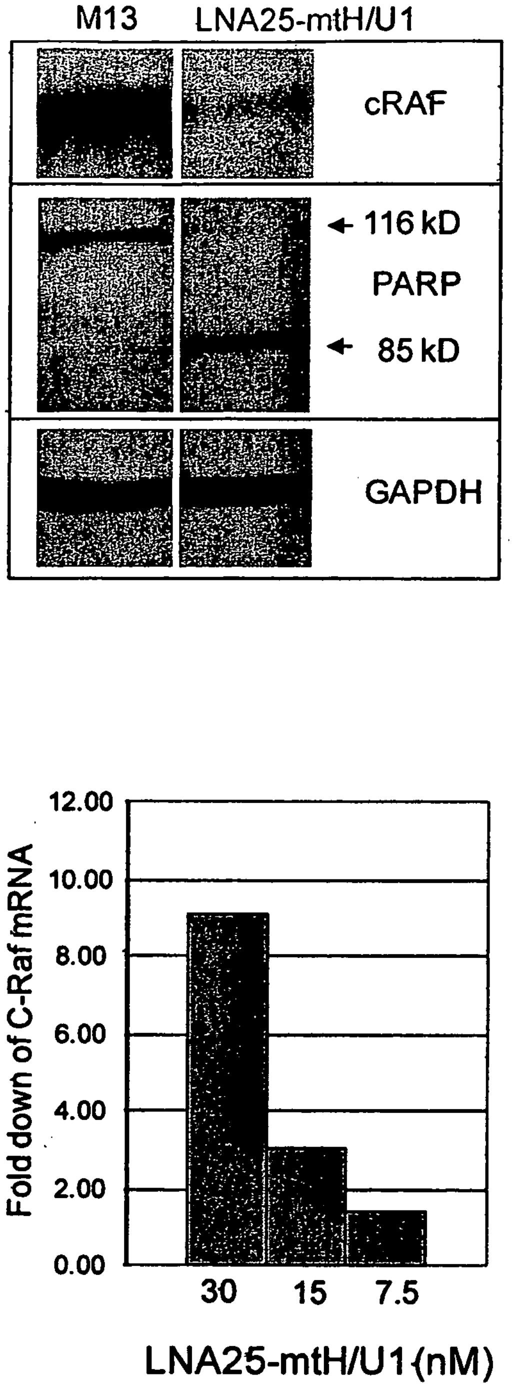
M13
LNA25-mtH/U1
cRAF
116 kD
PARP
85 kD
GAPDH
Fold down of C-Raf mRNA
12.00
10.00
8.00
6.00
4.00
2.00
0.00
30
15
7.5
LNA25-mtH/U1 (nM)

**Figure 15**

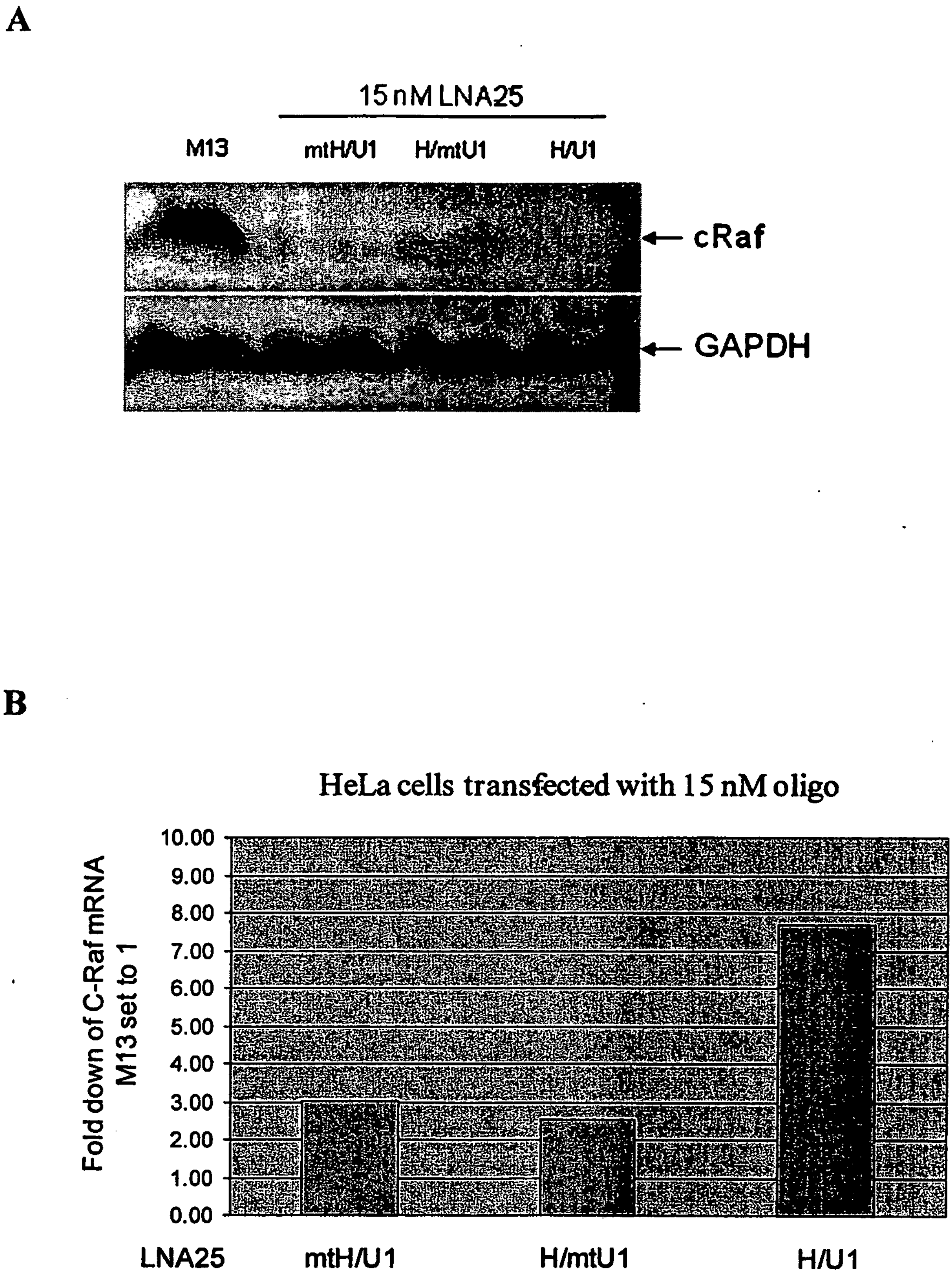
A
15 nM LNA25
M13
mtH/U1
H/mtU1
H/U1
cRaf
GAPDH
B
HeLa cells transfected with 15 nM oligo
Fold down of C-Raf mRNA
M13 set to 1
10.00
9.00
8.00
7.00
6.00
5.00
4.00
3.00
2.00
1.00
0.00
LNA25
mtH/U1
H/mtU1
H/U1

**Figure 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030082149 A
- US 20050043261 A
- US 20050118605 A
- US 5886165 A
- US 6140482 A
- US 5693773 A
- US 5856462 A
- US 5973136 A
- US 5929226 A
- US 6194598 A
- US 6172209 A
- US 6175004 A
- US 6166197 A
- US 6166188 A
- US 6160152 A
- US 6160109 A
- US 6153737 A
- US 6147200 A
- US 6146829 A
- US 6127533 A
- US 6124445 A
- US 4683195 A
- US 4800195 A
- US 4965188 A
- US 4897355 A
- US 4399498 A
- US 4235871 A
- US 4231877 A
- US 4224179 A
- US 4753788 A
- US 4673567 A
- US 4247411 A
- US 4814270 A
- US 5567434 A
- US 5552157 A
- US 5565213 A
- US 5738868 A
- US 5795587 A
- US 5922859 A
- US 6077663 A


### Non-patent literature cited in the description


- **Novina et al.** *Nature,* 2004, vol. 430, 161-4
- **Eckner et al.** *EMBO J.,* 1991, vol. 10, 3513-3522
- **Sachs et al.** *J. Biol. Chem.,* 1993, vol. 268, 22955-8
- **Zhao et al.** *Microbiol. Mol. Biol. Rev.,* 1999, vol. 63, 405-445
- **Proudfoot et al.** *Cell,* 2002, vol. 108, 501-12
- **Proudfoot et al.** *Nature,* 1976, vol. 263, 211-4
- **Sheets et al.** *Nucl. Acids Res.,* 1990, vol. 18, 5799-805
- **Beaudoing et al.** *Gen. Res.,* 2000, vol. 10, 1001-1010
- **Tian et al.** *Nuc. Acids Res.,* 2005, vol. 33, 201-12
- **Chen et al.** *Nuc. Acids Res.,* 1995, vol. 23, 2614-2620
- **Boelens et al.** *Cell,* 1993, vol. 72, 881-892
- **Gunderson et al.** *Cell,* 1994, vol. 76, 531-541
- **Gunderson et al.** *Genes Dev.,* 1997, vol. 11, 761-773
- **Guan et al.** *Mol. Cell. Biol.,* 2003, vol. 23, 3163-3172
- **Furth et al.** *Mol. Cell. Biol.,* 1994, vol. 14, 5278-5289
- **Will et al.** *Curr. Opin. Cell Biol.,* 1997, vol. 9, 320-8
- **Gunderson et al.** *Mol. Cell,* 1998, vol. 1, 255-264
- **Beckley et al.** *Mol. Cell Biol.,* 2001, vol. 21, 2815-25
- **Sajic et al.** *Nuc. Acids Res.,* 2007, vol. 35, 247-55
- **Liu et al.** *Nuc. Acids Res.,* 2002, vol. 30, 2329-39
- **Fortes et al.** *Proc. Natl. Acad. Sci.,* 2003, vol. 100, 8264-8269
- **Ittig et al.** *Nuc. Acids Res.,* 2004, vol. 32, 346-53
- **Kauppinen et al.** *Drug Discov. Today Tech.,* 2005, vol. 2, 287-290
- **Orum et al.** *Letters Peptide Sci.,* 2004, vol. 10, 325-334
- **Hunziker ; Leumann.** Nucleic Acid Analogues: Synthesis and Properties, in Modern Synthetic Methods. VCH, 1995, 331-417

- **Mesmaeker et al.** Novel Backbone Replacements for Oligonucleotides, in Carbohydrate Modifications in Antisense Research. ACS, 1994, 24-39
- **Kyriakopoulou et al.** *RNA,* 2006, vol. 12, 1603-11
- **Abad et al.** Requirements for gene silencing mediated by U1snRNA binding to a target sequence. *Nucleic Acids Res,* 25 February 2008
- **Behr.** *Bioconjugate Chem,* 1994, vol. 5, 382-389
- **Lewis et al.** *PNAS,* 1996, vol. 93, 3176-3181
- **Myslinski et al.** *Nucl. Acids Res.,* 2001, vol. 29, 2502-09
- **Gunderson et al.** *Genes and Dev.,* 1997, vol. 11, 761-773
- **Elbashir et al.** *Nature,* 2001, vol. 411, 494-8
- **Monia et al.** *Nat Med.,* 1996, vol. 2, 668-75
- **Pfaffl, M.W.** *Nuc. Acid Res.,* 2001, vol. 29, 2002-2007
- **Kurreck et al.** *Nuc. Acids Res.,* 2002, vol. 30, 1911-8
- **Grünweller et al.** *Nuc. Acids Res.,* 2003, vol. 31, 3185-93
- **Lau et al.** *Oncogene,* 1998, vol. 16, 1899-902